# EUROPEAN PATENT APPLICATION

(11) **EP 3 231 434 A1**
(43) Date of publication of application: **18.10.2017**
(21) Application number: 16165348.0
(22) Date of filing: 14.04.2016
(51) Int. Cl.: A61K 35/16, A61P 25/16

(54) **METHOD OF TREATMENT OF PARKINSONISM**

(71) Applicant: Fundacio Centre de Regulacio Genomica, 08003 Barcelona (ES); Institució Catalana De Recerca I Estudis Avançats (ICREA), 08010 Barcelona (ES)
(72) Inventor: COSMA, Maria Pia, E-08003 Barcelona (ES); ALTARCHE XIFRO, Wassim, E-08003 Barcelona (ES)
(74) Representative: ABG Patentes, S.L.

(57) **Abstract**

The invention relates to a cell selected from the group consisting of a hematopoietic stem cell (HSC), a hematopoietic progenitor cell (HPC), and a mesenchymal stem cell (MSC), or a cell population comprising any combination thereof, for use in the treatment of Parkinsonism in a subject, and to pharmaceutical compositions and kits for use in the treatment of Parkinsonism.

SEQUENCE LISTING

<110>  FUNDACIÓ CENTRE DE REGULACIÓ GENÒMICA
       INSTITUCIÓ CATALANA DE RECERCA I ESTUDIS AVANÇATS (ICREA)

<120>  METHOD OF TREATMENT OF PARKINSONISM

<130>  P12567EP00

<160>  40

<170>  PatentIn version 3.5

<210>  1
<211>  22
<212>  DNA
<213>  Artificial Sequence

<220>
<223>  Forward primer beta-Actin

<400>  1
acgttgacat ccgtaaagac ct                                                22

<210>  2
<211>  22
<212>  DNA
<213>  Artificial Sequence

<220>
<223>  Reverse primer beta actin

<400>  2
gcagtaatct ccttctgcat cc                                                22

<210>  3
<211>  19
<212>  DNA
<213>  Artificial Sequence

<220>
<223>  Forward primer CD34

<400>  3
tggtacttcc agggatgct                                                    19

<210>  4
<211>  20
<212>  DNA
<213>  Artificial Sequence

<220>
<223>  Reverse primer CD34

<400>  4
tgggtagctc tctgcctgat                                                   20

<210>  5
<211>  20

<212>  DNA
<213>  Artificial Sequence

<220>
<223>  Forward primer Oct4

<400>  5
cgtggagact ttgcagcctg                                                    20


<210>  6
<211>  24
<212>  DNA
<213>  Artificial Sequence

<220>
<223>  Reverse primer Oct4

<400>  6
gcttggcaaa ctgttctagc tcct                                               24


<210>  7
<211>  21
<212>  DNA
<213>  Artificial Sequence

<220>
<223>  Forward primer GFAP

<400>  7
ggagacgcat cacctctgcg c                                                  21


<210>  8
<211>  20
<212>  DNA
<213>  Artificial Sequence

<220>
<223>  Reverse primer GFAP

<400>  8
agaaatccac ccgggtcggg                                                    20


<210>  9
<211>  14
<212>  DNA
<213>  Artificial Sequence

<220>
<223>  Forward primer Nestin

<400>  9
tggaagtggc taca                                                          14


<210>  10
<211>  16
<212>  DNA
<213>  Artificial Sequence

<220>
<223>  Reverse primer Nestin

<400>  10
tcagcttggg gtcagg                                                       16


<210>  11
<211>  21
<212>  DNA
<213>  Artificial Sequence

<220>
<223>  Forward primer Axin2

<400>  11
gggagcagtt ttgtggcagc a                                                 21


<210>  12
<211>  23
<212>  DNA
<213>  Artificial Sequence

<220>
<223>  Reverse primer Axin2

<400>  12
agggtcctgg gtaaatgggt gag                                               23


<210>  13
<211>  21
<212>  DNA
<213>  Artificial Sequence

<220>
<223>  Forward primer Wnt5a

<400>  13
tgcggagaca aacatcgact a                                                 21


<210>  14
<211>  20
<212>  DNA
<213>  Artificial Sequence

<220>
<223>  Reverse primer Wnt5a

<400>  14
aagttcatga ggatgcgtgc                                                   20


<210>  15
<211>  20
<212>  DNA
<213>  Artificial Sequence

<220>
<223>  Forward primer Fzd1

<400>  15
gtcggtttca aggctcctcc                                                   20


<210>  16
<211>  20
<212>  DNA
<213>  Artificial Sequence

<220>
<223>  Reverse primer Fzd1

<400>  16
cctccaacgg gtttctaggc                                                   20


<210>  17
<211>  24
<212>  DNA
<213>  Artificial Sequence

<220>
<223>  Forward primer Runx1

<400>  17
cacctgtctc tgcatcgcag gact                                              24


<210>  18
<211>  24
<212>  DNA
<213>  Artificial Sequence

<220>
<223>  Reverse primer Runx1

<400>  18
ccatccgtga cagatacgca cctc                                              24


<210>  19
<211>  23
<212>  DNA
<213>  Artificial Sequence

<220>
<223>  Forward primer Sox2

<400>  19
caggagaacc ccaagatgca caa                                               23


<210>  20
<211>  22
<212>  DNA
<213>  Artificial Sequence

<220>
<223>  Reverse primer Sox2

<400>  20
aatccgggtg ctccttcatg tg                                                22

<210>  21
<211>  27
<212>  DNA
<213>  Artificial Sequence

<220>
<223>  Forward primer Olig2

<400>  21
caaatctaat tcacattcgg aaggttg                                          27


<210>  22
<211>  21
<212>  DNA
<213>  Artificial Sequence

<220>
<223>  Reverse primer Olig2

<400>  22
gacgatgggc gactagacac c                                                21


<210>  23
<211>  19
<212>  DNA
<213>  Artificial Sequence

<220>
<223>  Forward primer MAP2

<400>  23
ttggtgccga gtgagaaga                                                   19


<210>  24
<211>  20
<212>  DNA
<213>  Artificial Sequence

<220>
<223>  Reverse primer MAP2

<400>  24
gtctggcagt ggttggttaa                                                  20


<210>  25
<211>  21
<212>  DNA
<213>  Artificial Sequence

<220>
<223>  Forward primer FoxA2

<400>  25
ccctacgcca acatgaactc g                                                21


<210>  26
<211>  20

<212>  DNA
<213>  Artificial Sequence

<220>
<223>  Reverse primer FoxA2

<400>  26
gttctgccgg tagaaaggga                                                    20


<210>  27
<211>  20
<212>  DNA
<213>  Artificial Sequence

<220>
<223>  Forward primer NeuN

<400>  27
ggcaatggtg ggactcaaaa                                                    20


<210>  28
<211>  18
<212>  DNA
<213>  Artificial Sequence

<220>
<223>  Reverse primer NeuN

<400>  28
gggacccgct ccttcaac                                                      18


<210>  29
<211>  23
<212>  DNA
<213>  Artificial Sequence

<220>
<223>  Forward primer Wnt1

<400>  29
ggtttctact acgttgctac tgg                                                23


<210>  30
<211>  21
<212>  DNA
<213>  Artificial Sequence

<220>
<223>  Reverse primer Wnt1

<400>  30
ggaatccgtc aacaggttcg t                                                  21


<210>  31
<211>  20
<212>  DNA
<213>  Artificial Sequence

<220>
<223>  Forward primer Caspase-9

<400>  31
tgggtctcgg cgggatcagg                                                    20


<210>  32
<211>  20
<212>  DNA
<213>  Artificial Sequence

<220>
<223>  Reverse primer Caspase-9

<400>  32
gcgtgtgctg agccctgagg                                                    20


<210>  33
<211>  20
<212>  DNA
<213>  Artificial Sequence

<220>
<223>  Forward primer GATA-4

<400>  33
ggcctctacc acaagatgaa                                                    20


<210>  34
<211>  20
<212>  DNA
<213>  Artificial Sequence

<220>
<223>  Reverse primer GATA-4

<400>  34
actgccagac taccaccacc                                                    20


<210>  35
<211>  23
<212>  DNA
<213>  Artificial Sequence

<220>
<223>  Forward primer Brachyury

<400>  35
gcttcaagga gctaactaac gag                                                23


<210>  36
<211>  22
<212>  DNA
<213>  Artificial Sequence

<220>
<223>  Reverse primer Brachyury

<400>  36
ccagcaagaa agagtacatg gc                                                22


<210>  37
<211>  19
<212>  DNA
<213>  Artificial Sequence

<220>
<223>  Forward primer Sox7

<400>  37
cagcaagatg ctgggaaag                                                    19


<210>  38
<211>  21
<212>  DNA
<213>  Artificial Sequence

<220>
<223>  Reverse primer Sox7

<400>  38
tgcatcatcc acatagggtc t                                                 21


<210>  39
<211>  20
<212>  DNA
<213>  Artificial Sequence

<220>
<223>  Forward primer BMP4

<400>  39
gagggatctt taccggctcc                                                   20


<210>  40
<211>  23
<212>  DNA
<213>  Artificial Sequence

<220>
<223>  Reverse primer BMP4

<400>  40
gttgaagagg aaacgaaaag cag                                               23

## Description

### FIELD OF THE INVENTION

This invention relates to the field of cell-based treatment of Parkinsonism.

### BACKGROUND OF THE INVENTION

Parkinsonism is a clinical syndrome manifested by a combination of the following cardinal features: tremor, bradykinesia, rigidity, loss of postural reflexes, flexed posture and freezing. The most common cause of Parkinsonism is Parkinson's disease (approximately 85% of all Parkinsonism cases). Other causes of Parkinsonism such as multisystem degenerations, hereditary and secondary (acquired/symptomatic) Parkinsonism, have a strong resemblance to Parkinson's disease, which suggests that the affected pathways may be in part similar or overlapping especially those related to dopaminergic *substantia nigra* pars compacta (SNpc) degeneration.

Parkinson's disease is one of the most common neurodegenerative disorders, and it affects about 1% of the population above the age of 60 years. The average onset of Parkinson's disease is at the age of 60, but can also occur in younger people ('Young Onset Parkinson's disease'; ≤ 60 years old; approximately 5% of the people). Parkinson's disease occurs in about 50% more in men than in women.

Currently there is no cure for Parkinson's disease, but medication and therapy are used to treat its symptoms. The goal of medical management of Parkinson's disease is to provide control of signs and symptoms for as long as possible while minimizing adverse effects.

Stem cell treatments for Parkinson's disease are still in the early stages of development. Some of the most important recent advances include work on methods for making dopamine-producing neurons in the lab; research on how to improve the effectiveness of transplants and avoid side effects; and studies investigating how the disease develops and how cells can help with the development of new drugs to stop this.

Cellular plasticity in human tissues can be modulated after fusion of bone marrow (BM)-derived cells with a variety of cells, such as neurons, hepatocytes, cardiomyocytes, and gut cells.

Current cell-therapy strategies in Parkinson's disease models are based on the transplantation of a variety of different cell types into the striatum with the aim of obtaining dopamine release (Gaillard and Jaber, 2011). Release of dopamine has been shown to occur directly from grafted neural stem cells obtained after differentiation of human embryonic stem cells (Kang et al., 2014). However, the transplanted cells usually do not integrate into the existing basal ganglia circuit, and they are only likely to function as dopamine producers (Gaillard and Jaber, 2011).

Therefore, there is the need to provide an effective method for treating Parkinson's Disease and other causes of Parkinsonism which are characterized by a degeneration of dopaminergic neurons.

### BRIEF SUMMARY OF THE INVENTION

In a first aspect, the invention relates to a cell selected from the group consisting of a hematopoietic stem cell (HSC), a hematopoietic progenitor cell (HPC), and a mesenchymal stem cell (MSC), or a cell population comprising any combination thereof, for use in the treatment of Parkinsonism in a subject.

In a second aspect, the invention relates to a pharmaceutical composition selected from the group consisting of:
1) a pharmaceutical composition comprising a cell selected from the group consisting of a hematopoietic stem cell (HSC), a hematopoietic progenitor cell (HPC), and a mesenchymal stem cell (MSC), or a cell population comprising any combination thereof, and a pharmaceutically acceptable carrier;
2) a pharmaceutical composition comprising a cell selected from the group consisting of a hematopoietic stem cell (HSC), a hematopoietic progenitor cell (HPC), and a mesenchymal stem cell (MSC), or a cell population comprising any combination thereof, wherein the Wnt/β-catenin signaling pathway of said cells is activated, and a pharmaceutically acceptable carrier; and
3) a pharmaceutical composition comprising a cell selected from the group consisting of a hematopoietic stem cell (HSC), a hematopoietic progenitor cell (HPC), and a mesenchymal stem cell (MSC), or a cell population comprising any combination thereof, a Wnt/β-catenin signalling pathway activator or an inhibitor of a Wnt/β-catenin signalling pathway repressor, and a pharmaceutically acceptable carrier,
for use in the treatment of Parkinsonism.

### BRIEF DESCRIPTION OF THE DRAWINGS

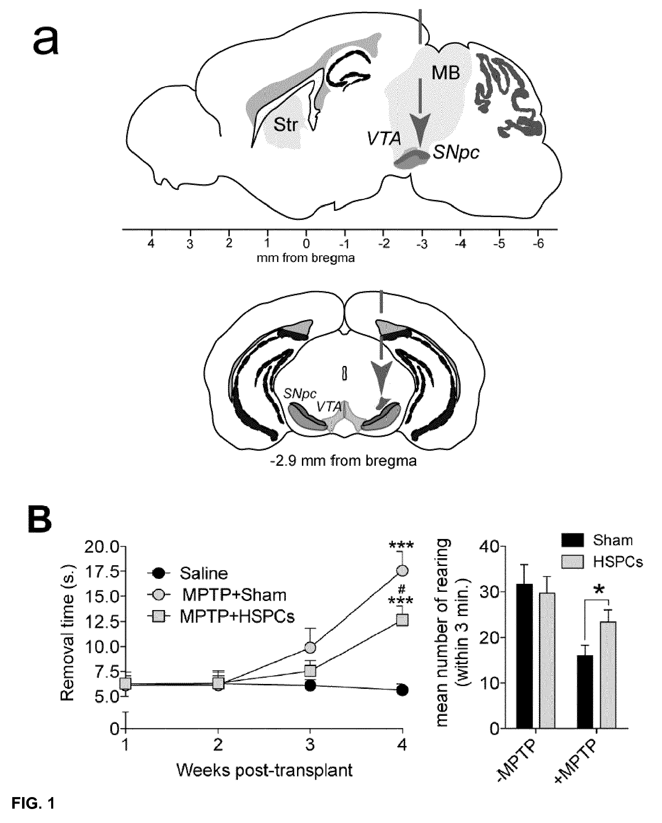

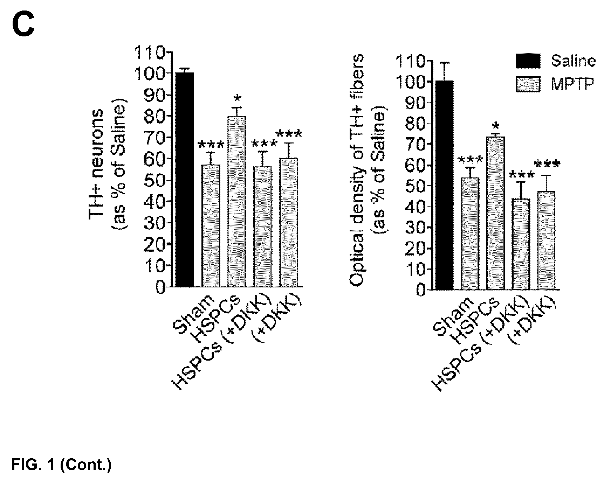

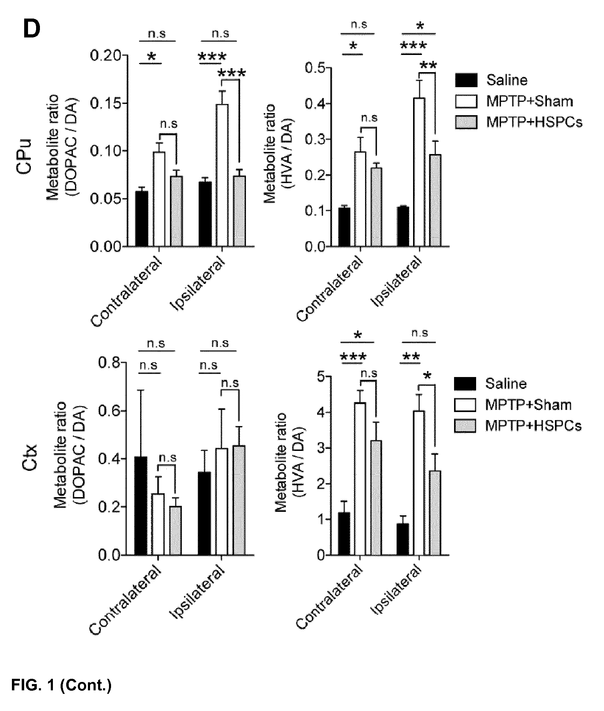

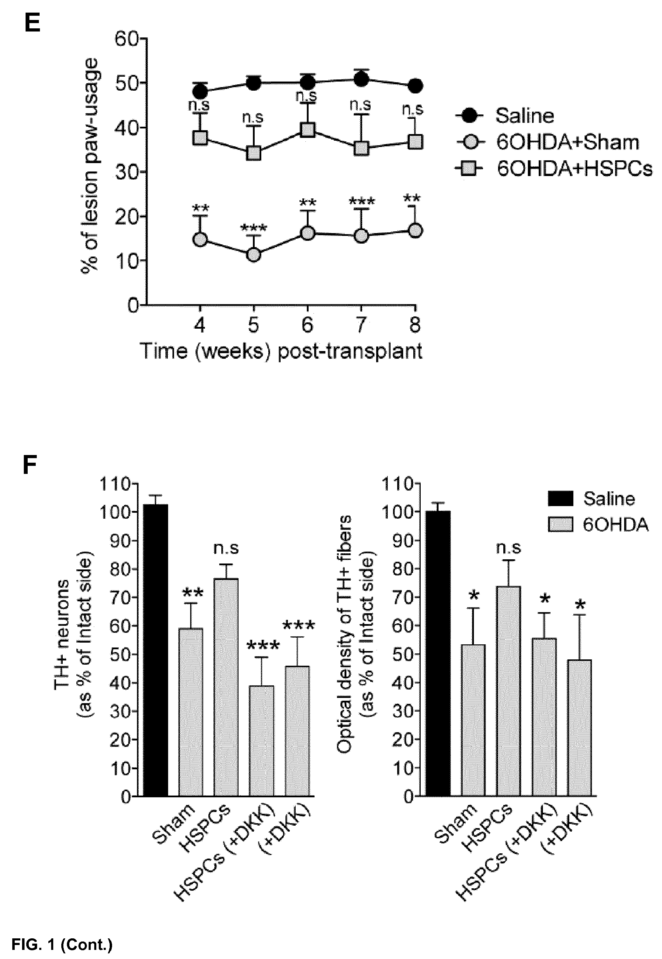

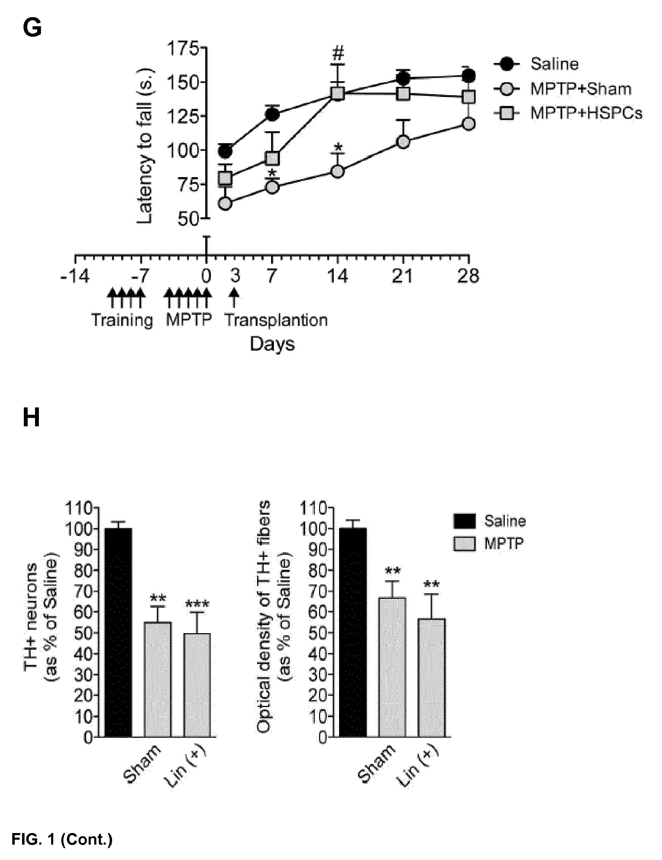

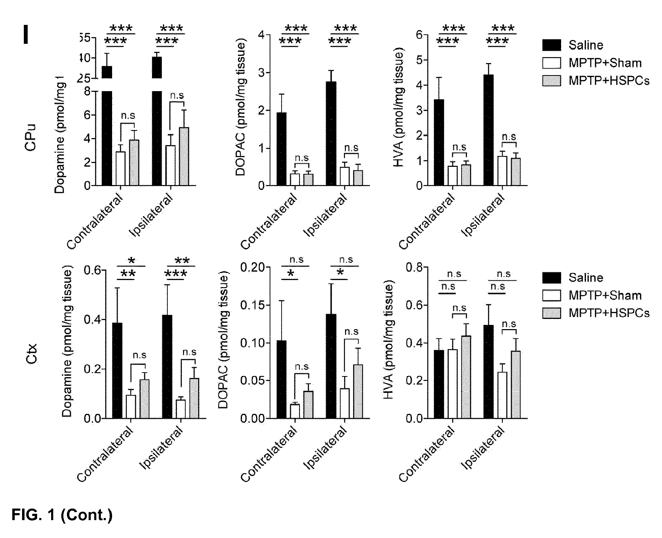

**Figure 1****. Hematopoietic stem and progenitor cells (HSPC) transplantation functionally rescued dopaminergic neurons.** (A) Representation of a sagittal brain section and a coronal section, indicating the site of HSPC transplantation. Midbrain area (MB), substantia nigra pars compacta (SNpc), ventral tegmental area (VTA), and striatum (Str). (B) MPTP+Sham (*n*=8*)* and MPTP+HSPCs (*n*=11) transplanted mice were evaluated for time to stimulus removal (from 1 to 4 weeks after transplantation) and for spontaneous activity upon quantification of the number of rears (4 weeks after transplantation). (C) Quantification of the number of TH+ neurons in the midbrain by stereology, and TH+ striatal fibers by optical densitometry, in MPTP+Sham (*n*=11), MPTP+HSPCs (*n*=11), MPTP+HSPCs (DKK) (*n*=7), and MPTP+DKK (*n*=7) transplanted mice, relative to Saline (*n*=8) transplanted mice. (D) Striatal DA turnover, as determined by the DOPAC/DA and HVA/DA ratios. DA, dopamine; CPu, striatum/ caudate-putamen; Ctx, cortex. (E) 6OHDA+Sham (n=6) and 6OHDA+HSPCs (*n*=10) transplanted mice were assessed for forepaw akinesia using the cylinder test. (F) Percentage of TH+ neurons and fibers loss in the 6OHDA+Sham (n=9), 6OHDA+HSPCs (n=10), 6OHDA+HSPCs (DKK) (n=8) and 6OHDA+DKK (n=7) transplanted mice, relative to intact (Saline) side. DKK, Dickkopf-related protein 1 or DKK1. Data are means ±s.e.m. Statistical analysis is based on one-way ANOVA followed by Bonferroni's multiple comparison post-hoc tests. *P <0.05; **P <0.01; ***P <0.001 compared with Saline; n.s, not significant; s, seconds.
   **Characterization of the functional rescue of the MPTP mice.** (G) Mice were tested using the rotarod test 7, 14, 21, and 28 days after HSPCs transplantation. Data are means ±s.e.m for the time spent on the rotarod before falling off. Saline (*n*=4), MPTP+Sham (*n*=7), and MPTP+HSPCs (*n*=9). Two-way ANOVA: **P* <0.05 for days 7 and 14 compared with Saline. ^{#} *P* <0.05 for day 14 compared with MPTP+Sham. (H) Quantification of the number of TH+ neurons in the midbrain by stereology, and TH+ striatal fibers by optical densitometry, in MPTP+Sham (n=6) and MPTP+Lin(+) (n=6) mice relative to Saline (n=6) mice. Lin(+), Lineage positive cells. Data are means ± s.e.m. Statistical analysis is based on one-way ANOVA followed by Bonferroni's multiple comparison post-hoc tests. **P <0.01; ***P <0.001 compared with Saline. (I) HPLC analysis for the levels of dopamine and its metabolites DOPAC and HVA in the striatum/ caudate-putamen (CPu) and cortex (Ctx) of Saline (n=4) MPTP+Sham (n=7), and MPTP+HSPCs (n=9) mice. The analysis was performed 4 weeks after transplantation. Data are means ±s.e.m. Statistical analysis is based on one-way ANOVA followed by Bonferroni's multiple comparison post-hoc tests. *P<0.05; **P<0.01; ***P<0.001 compared with Saline; n.s: not significant; s, seconds.

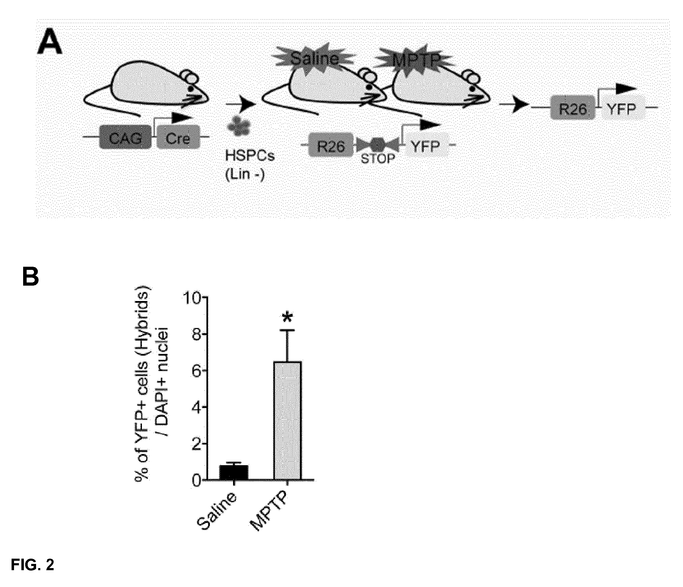

**Figure 2****. Hematopoietic stem and progenitor cells spontaneously fuse with brain somatic cells *in vivo.*** (A) Outline of the experimental design to detect cell-fusion events. Hybrids were detected by the Cre/loxP system, where Cre-recombinase is under the control of the CAG promoter. Mice were transplanted with lineage-depleted (Lin-) HSPCs after saline and MPTP treatment. Cre-mediated excision of the Stop codon in floxed-loxP transgenic mouse (R26Y), allowing yellow fluorescent protein (YFP) expression. (B) Quantification of the YFP-immunopositive cells found per DAPI+ nucleus in the saline and MPTP-treated mice. Data are means ±s.e.m (5 sections/mouse; n=6). Statistical analysis is based on Student's t-tests. *P <0.05.

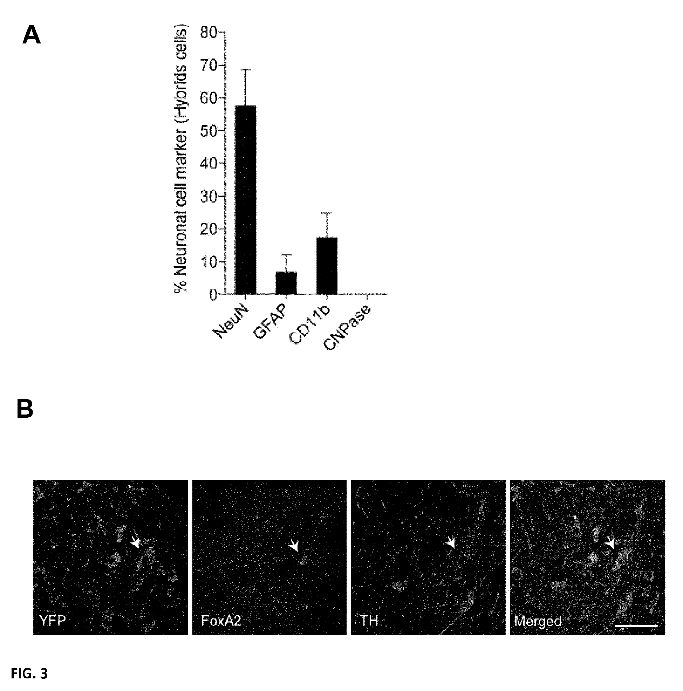

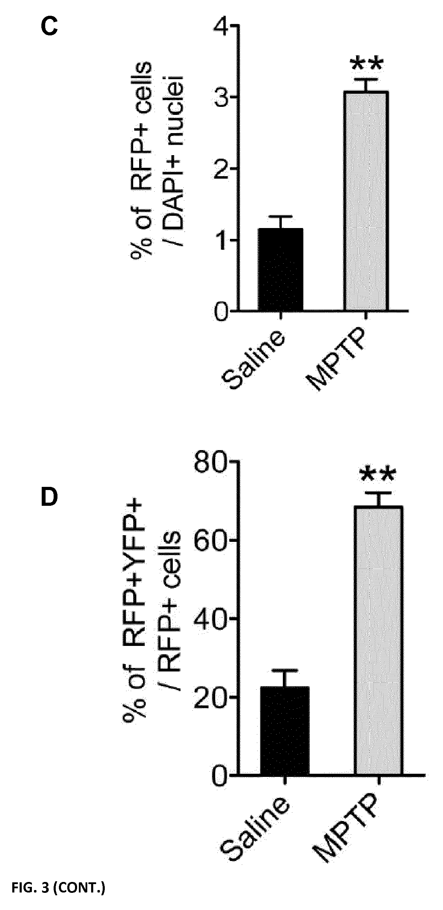

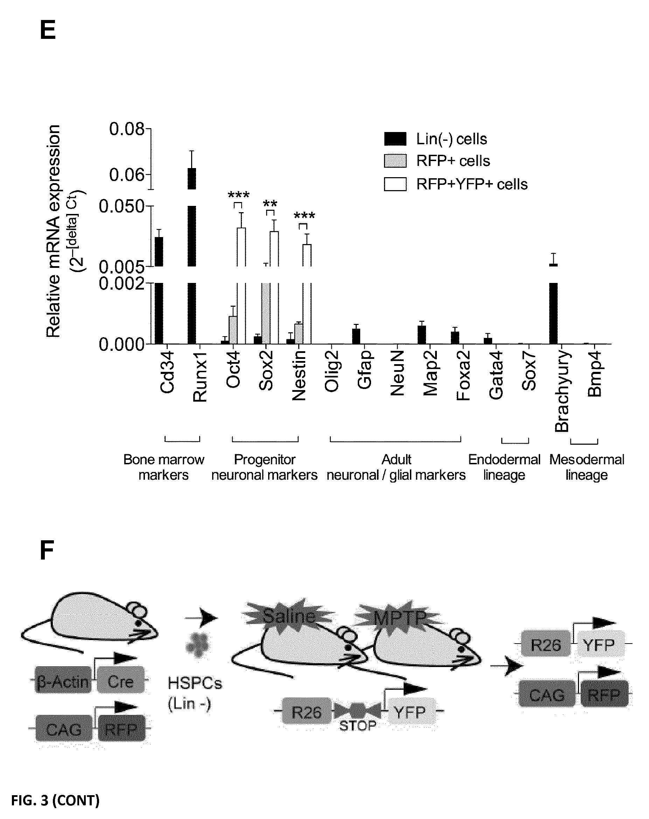

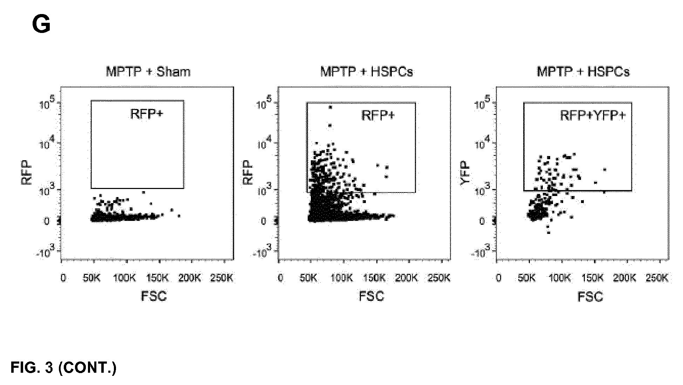

**Figure 3****. Characterization of hybrids formed *in vivo.***
   (A-C) R26Y mice transplanted as described in the legend to Figure 1 A were sacrificed 24 h after transplantation (*n*=6). (A) Cell number analysis of the YFP-immunopositive cells showing co-localization with NeuN, GFAP, CD11b, and CNPase (5 sections/mouse; *n*=6). (B) Immunostaining showing co-localization of YFP-immunopositive cells with TH and FoxA2 at the single-cell level. Arrows indicate co-localization. Scale bar: 50 µm. (C) Quantification of the RFP+ cells in the saline and MPTP-treated mice (6 sections/mouse; n=4). (D) Percentage of the double-positive cells (RFP+YFP+) in the saline and MPTP-treated mice (n=4). (E) qRT-PCR analysis of the expression of the indicated genes in the RFP+YFP+ and RFP+ FACS-sorted cells (3 mice/group). In all the immunostaining sections, the cell nuclei were counterstained with DAPI. Data are means ±s.e.m. Statistical analysis is based on Student's t-tests. **P <0.01; ***P <0.001.
   **Flow activated cell sorting and genome-wide analysis of hybrid cells (RFP+YFP+) and non-hybrid cells (RFP+).** (F) Schematic representation of the transgenes expressed by the mouse lines used to detect cell fusion events. Cre-recombinase was under the control of the β-Actin promoter, and RFP under the CAG promoter. R26Y mice were transplanted with lineage depleted (Lin-) HSPCs after saline and MPTP treatments. (G) FACS plots. Left: RFP+ cells in the Sham control brain (MPTP+Sham). Middle: surviving RFP+ cells in an MPTP brain 1 week after transplantation (MPTP+HSPCs). Right: double-positive (RFP+YFP+) cells gated on RFP+ cells. Cells were dissociated from three mouse brains and FACS sorted (n=2). FACS analysis was based on endogenous RFP and YFP fluorescence. Dead cells were excluded by gating on propidium-iodide-labeled cells.

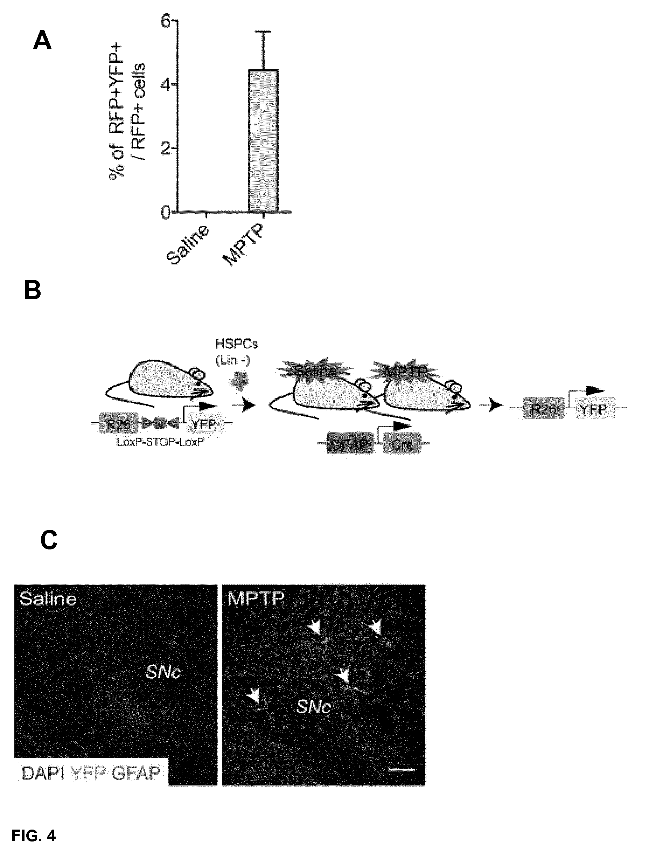

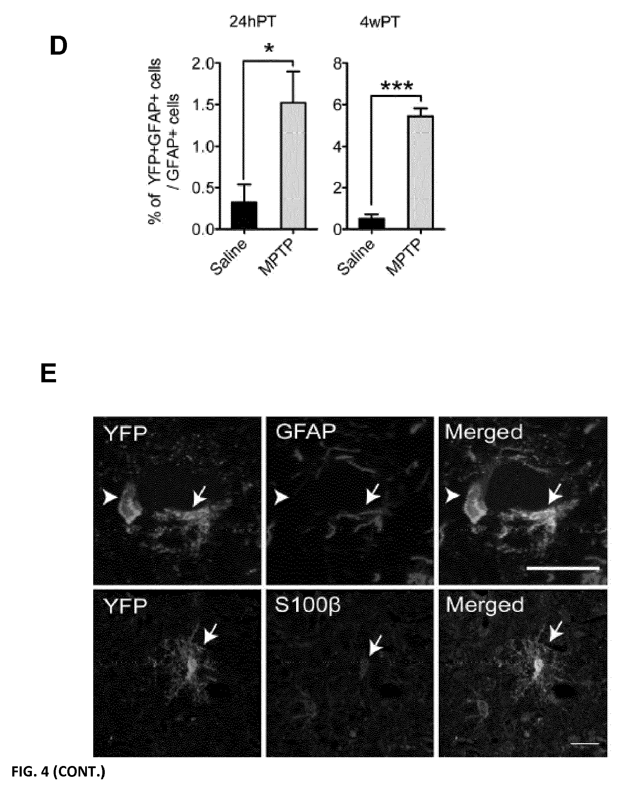

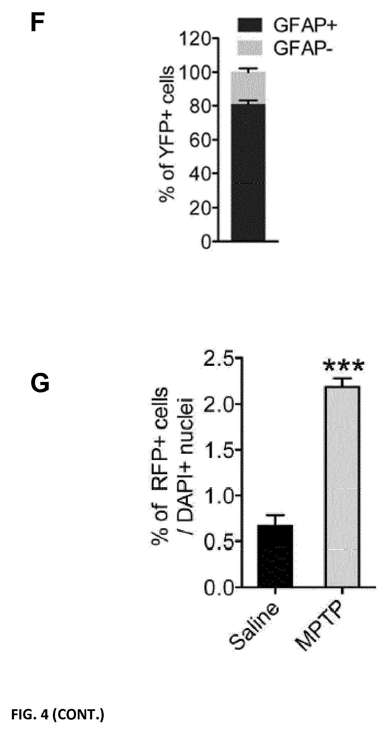

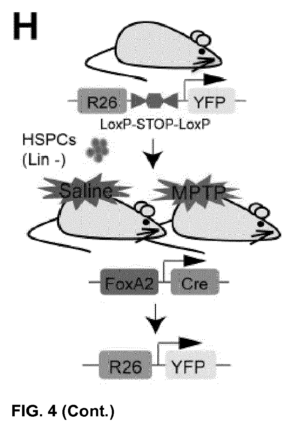

**Figure 4****. Hybrids can acquire features of GFAP-expressing astroglial cells 4 weeks after transplantation.**

   R26Y mice transplanted as described in the legend to Figure 1A were sacrificed 4 weeks after transplantation (n=4). (A) Quantification of RFP+YFP+ cells in the saline and MPTP-treated mice (*n*=4). (B) Schematic representation of the transplantation scheme and of mouse strains used in (C-F). Cre recombinase is under the control of the GFAP promoter, allowing identification of astroglial-derived hybrids. Mice were sacrificed 4 weeks after transplantation (*n*=4). (C) Representative immunostaining sections showing YFP+ cells co-expressing the astroglial marker (GFAP). SNc, substantia nigra pars compacta. Arrows indicate hybrids. Scale bar: 100 µm. (D) Cell fusion quantified 24 hours (24hPT) and 4 weeks (4wPT) after transplantation as the number of YFP+GFAP+ cells with respect to the total GFAP+ cells (6 sections/mouse; n=4). (E) YFP hybrids co-localize (arrows) with GFAP and S100β markers. Arrowheads indicate YFP+GFAP- cells. Scale bar: 20 µm. (F) Quantification of YFP+GFAP+ and YFP+GFAP- cells. (G) Quantification of RFP+ cells in saline and MPTP mice (n=4). ****P* <0.001. (H) Schematic representation of transplantation experiments and of mouse lines. Recipient mice were transgenic with Cre recombinase under the control of specific neuron midbrain transcription factor, the forkhead box protein A2 (FoxA2). The mice were sacrificed 4 weeks after transplantation.
   Data are means ±s.e.m. Statistical analysis is based on Student's *t*-tests. ****P* <0.001; n.s: not significant.

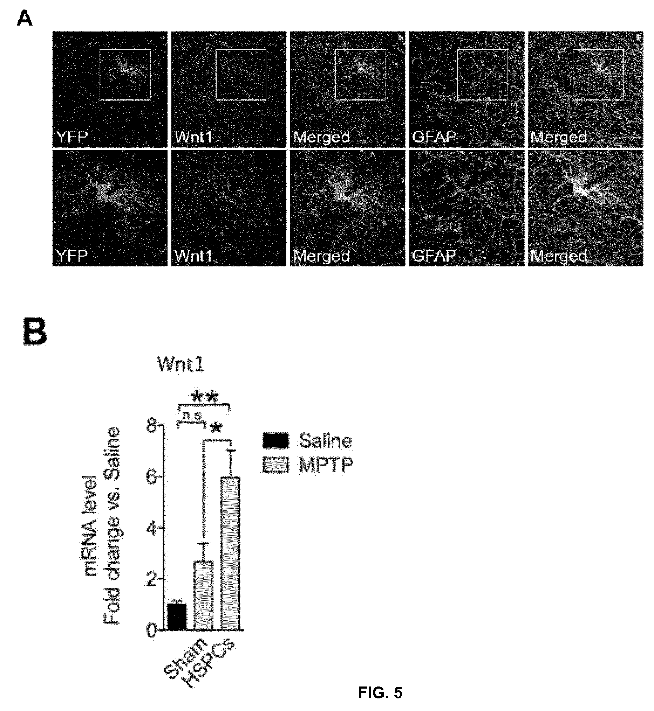

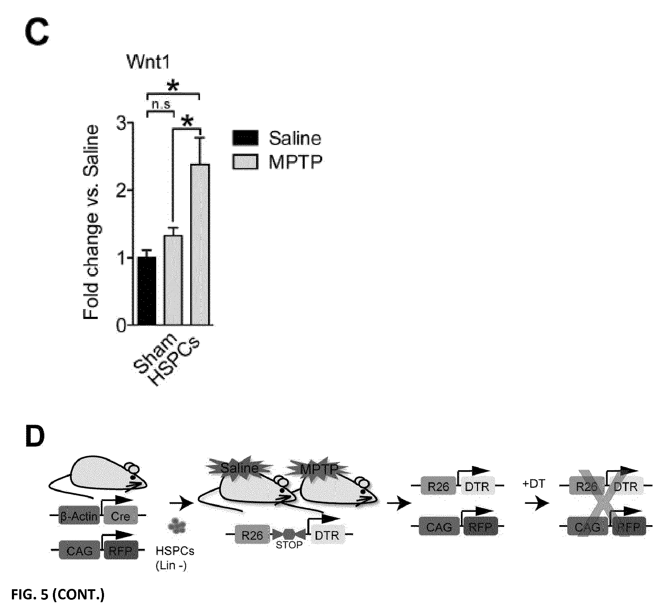

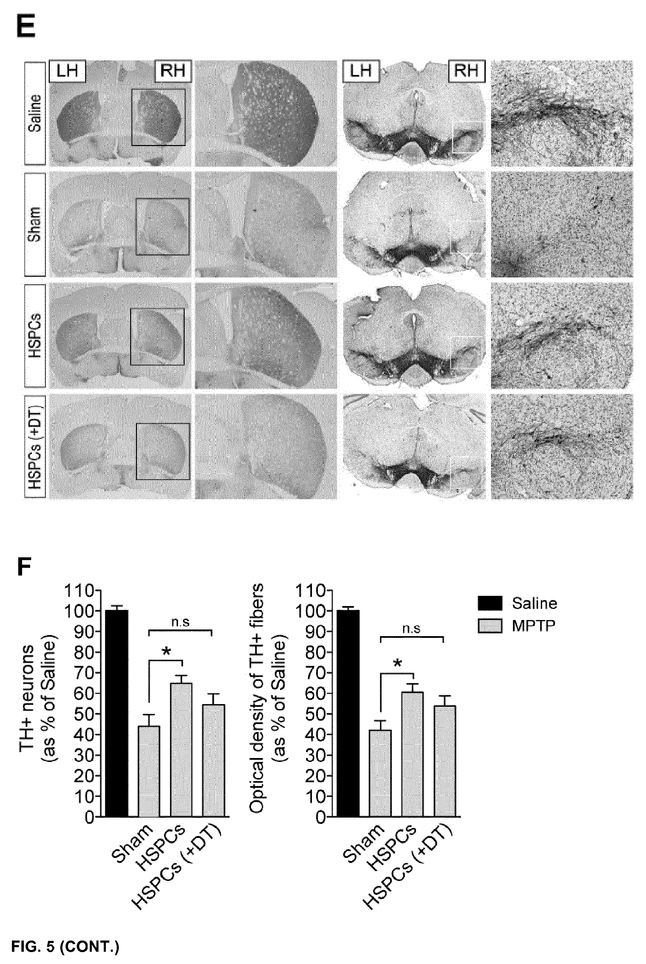

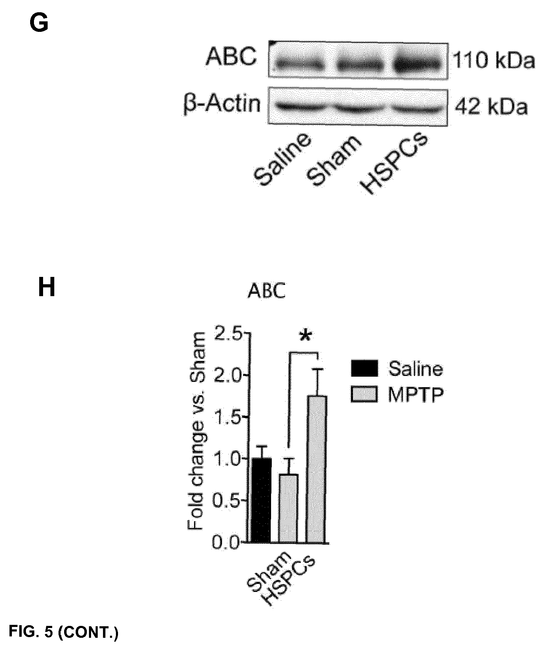

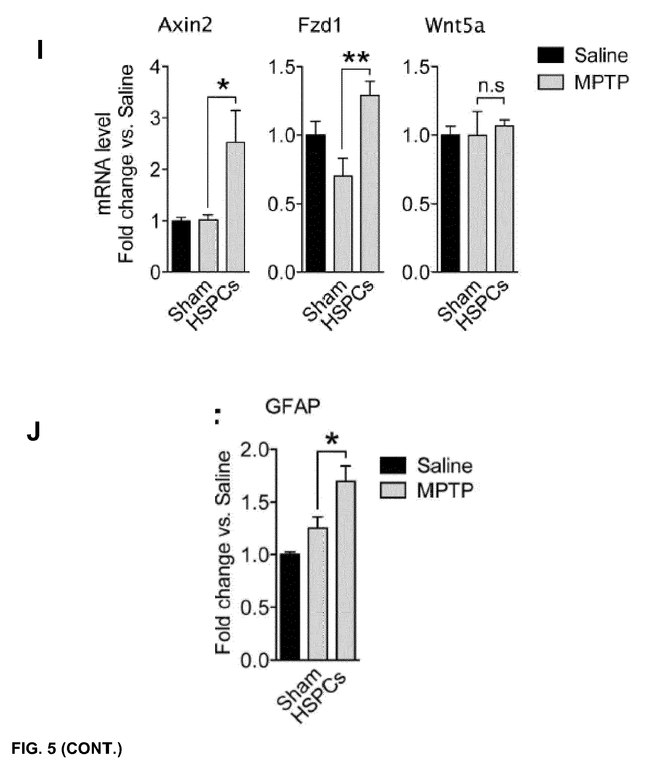

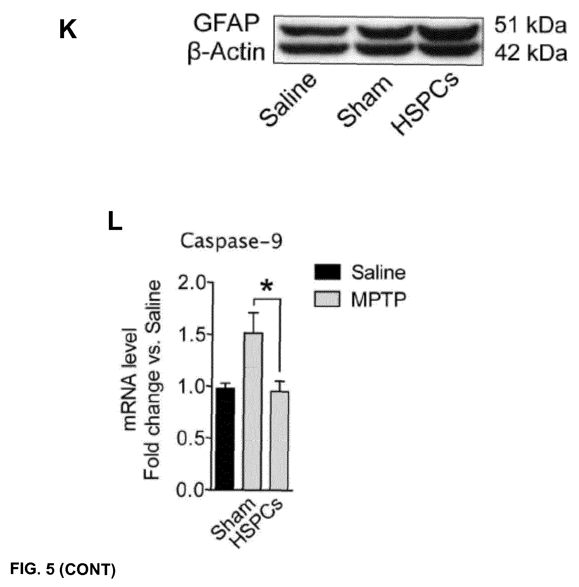

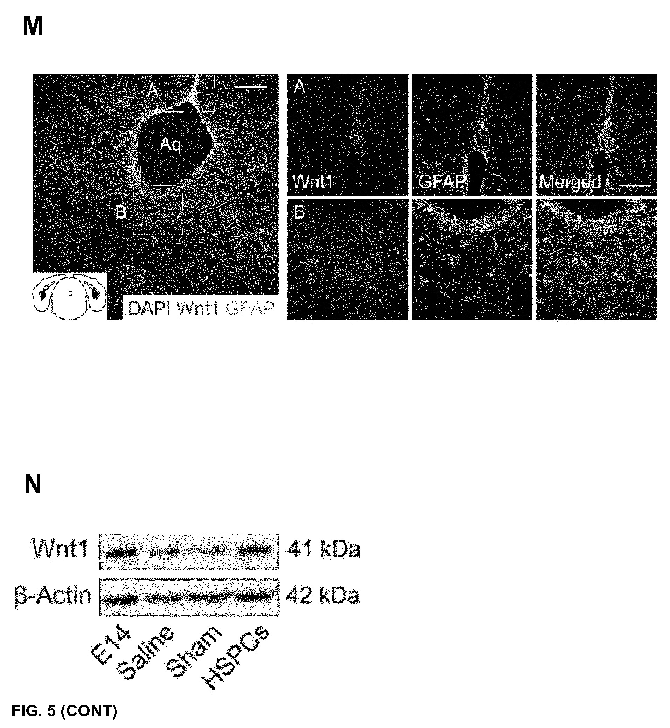

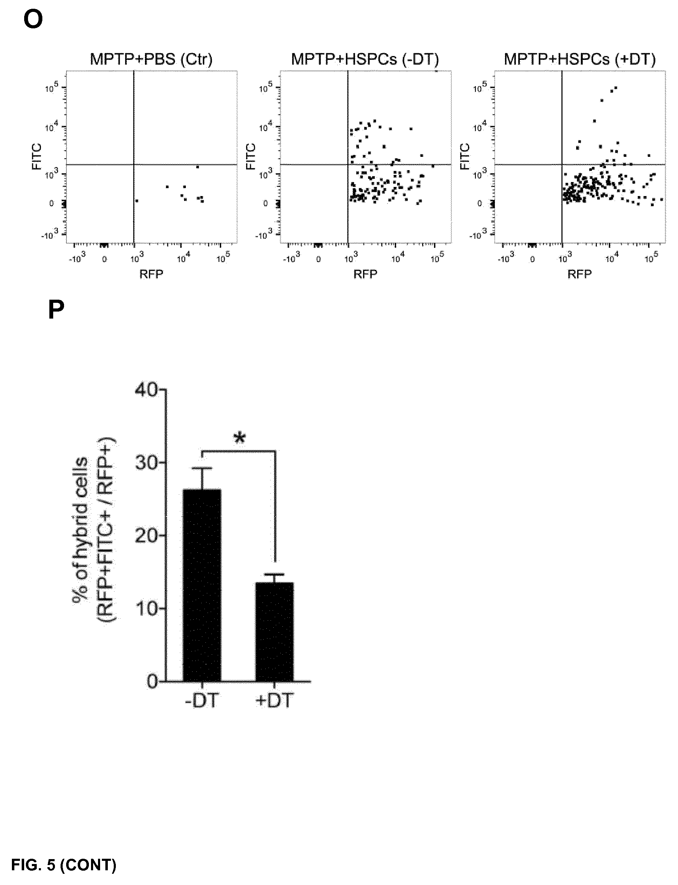

**Figure 5****. Newly generated hybrid-derived astroglia secrete Wnt1.**
   (A) Wnt1 protein co-localization with YFP+ hybrids and the astroglia marker GFAP in GFAP-cre transplanted mice sacrificed 4 weeks after transplantation. Scale bar: 50 µm. Representative higher magnification images are as indicated for the white boxes. In all immunostaining images, cell nuclei were counterstained with DAPI. (B, C) Quantification of Wnt1 mRNA (B) and protein (C) levels in midbrain homogenates of transplanted mice. The housekeeping gene and protein, β-actin, was used to normalize the qRT-PCR and Western blotting, respectively. Data are means ±s.e.m. for fold changes versus Saline mice, (n=4-6/group). Statistical analysis is based on one-way ANOVA followed by Bonferroni's multiple comparison post-hoc tests. *P <0.05; **P <0.01; n.s: not significant. (D) R26DTR mice transplanted as described in the legend to Figure 1A were sacrificed 4 weeks after transplantation. (E) Representative coronal sections of each group of mice (MPTP model) showing immunohistochemistry of endogenous TH in the striatum and midbrain. (F) Quantification of the number of TH+ neurons in the midbrain by stereology, and TH+ striatal fibers by optical densitometry, in MPTP+Sham (n=9), MPTP+HSPCs (n=10) and MPTP+HSPCs (DT) (n=9) mice relative to Saline (n=6) mice. DT, diphtheria toxin; RH, right hemisphere; LH, left hemisphere. Data are means ±s.e.m. Statistical analysis is based on one-way ANOVA followed by Bonferroni's multiple comparison post-hoc tests. *P <0.05 compared to Sham; n.s, not significant.
   **Wnt signaling activity and glial expression levels in HSPC-transplanted mice.**
   (G-M, O) Detection of proteins and mRNA from dissected ventral midbrains (VM) of MPTP (Sham) and MPTP-transplanted (HSPCs) mice, 21 days after the last MPTP injection. (G) Detection of the active β-catenin protein form (ABC) by Western blotting. (H) Quantification of active β-catenin (ABC) protein levels. (I) Axin2, Fzd1, and Wnt5a mRNA expression was carried out by real-time quantitative PCR. (J) GFAP mRNA levels. (K) GFAP protein by Western blotting. (L) Caspase-9 mRNA levels. (M) Representative immunostaining of Wnt1 protein expression and astrocytes (GFAP) for the caudal side of the adult midbrain. Aq, Aqueduct. Scale bar: 200 µm. Representative higher magnification images as indicated for A and B boxes. Scale bar: 100 µm. (N) Wnt1 protein levels. Embryonic (E-14) brain was used as the control tissue for Wnt1 protein expression. (O) FACS plots of the hybrid cells (RFP+FITC+) in the iDTR model after diphtheria toxin (DT) administration. FACS analysis was based on endogenous RFP fluorescence and used antibodies against DTR conjugated with FITC. (P) Quantification of percentages of RFP+FITC+ cells showing reduction in hybrids 7 days after cell transplantation (n=4/group). The housekeeping gene and protein, β-actin, was used to normalize for qRT-PCR and Western blotting, respectively. Data are means ±s.e.m. of fold-changes versus Saline mice, (n=4-6/group). Statistical comparisons were made using either Student's t-tests for two independent groups, or analysis of variance by one-way ANOVA followed by Bonferroni's multiple comparison post-hoc tests. *P <0.05; **P <0.01; n.s, not significant.

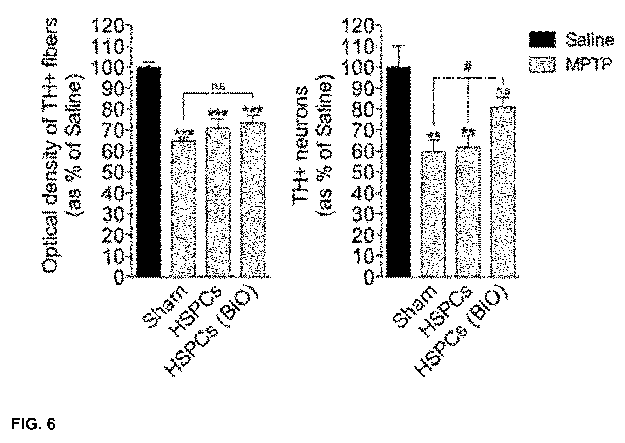

**Figure 6** **Wnt-activated HSPC transplantation rescued dopaminergic neurons in middle-aged mice.** Quantification of the number of TH+ neurons in the midbrain by stereology, and TH+ striatal fibers by optical densitometry, in MPTP+Sham (n=7), MPTP+HSPCs (n=7), MPTP+HSPCs (BIO) (n=7), transplanted mice, relative to Saline (n=4) transplanted mice. Data are means ±s.e.m. Statistical analysis is based on one-way ANOVA followed by Bonferroni's multiple comparison post-hoc tests. n.s, not significant; **P <0.01; ***P <0.001 compared with Saline; #P <0.05 compared with Sham or MPTP+HSPCs.

### DETAILED DESCRIPTION OF THE INVENTION

The inventors have observed that cellular functions in mouse and human tissues can be restored after fusion of bone marrow (BM)-derived cells with a variety of somatic cells. Here, after transplantation of hematopoietic stem and progenitor cells (HSPCs) in the substantia nigra pars compacta (SNpc) of two different mouse models of Parkinson's disease, the inventors significantly ameliorated the dopaminergic neuron loss and function. The inventors show fusion of transplanted HSPCs with neurons and with glial cells in the ventral midbrain of Parkinson's disease mice. Interestingly, the hybrids can undergo reprogramming *in vivo* and survived up to 4 weeks after transplantation, while acquiring features of mature astroglia. These newly generated astroglia produced Wnt1 and were essential for functional rescue of the dopaminergic neurons and subsequent functional recovery of the affected individuals. These data suggest that glial-derived hybrids produced upon fusion of transplanted HSPCs in the SNpc can rescue the Parkinson's disease phenotype via a niche-mediated effect, and can be exploited as an efficient cell-therapy approach.

### Cell or cell population

In a first aspect, the invention relates to a cell selected from the group consisting of a hematopoietic stem cell (HSC), a hematopoietic progenitor cell (HPC), and a mesenchymal stem cell (MSC), or a cell population comprising, consisting essentially of, or consisting of any combination thereof, for use in the treatment of Parkinsonism in a subject.

Alternatively, the invention relates to the use of a cell selected from the group consisting of a hematopoietic stem cell (HSC), a hematopoietic progenitor cell (HPC), and a mesenchymal stem cell (MSC), or a cell population comprising, consisting essentially of, or consisting of any combination thereof, for the preparation of a medicament for the treatment of Parkinsonism in a subject.

Alternatively, the invention relates to a method of treatment of Parkinsonism in a subject comprising administering a cell selected from the group consisting of a hematopoietic stem cell (HSC), a hematopoietic progenitor cell (HPC), and a mesenchymal stem cell (MSC), or a cell population comprising, consisting essentially of, or consisting of any combination thereof, to a subject in need thereof.

The term "population" of cells is any number of cells greater than 1, but is preferably at least 1x10³ cells, at least 1x10⁴ cells, at least 1x10⁵ cells, at least 1x10⁶ cells, at least 1x10⁷ cells, at least 1x10⁸ cells, or at least 1x10⁹ cells. In preferred embodiments of the invention, at least 50%, at least 55%, at least 60%, at least 70%, at least 80%, at least 85%, at least 90%, at least 91%, at least 92%, at least 93%, at least 94% or at least 95%, of the stem cells (% by cell number) in the cell population are hematopoietic stem cells (HSC), hematopoietic progenitor cells (HPC), and/or mesenchymal stem cells (MSC). In another preferred embodiment the cells of the cell population are at least 95% HSC and HPC, preferably 100% HSC and HPC.

Said cell population can be obtained, for example, from bone marrow, or, alternatively, by mixing HSCs, hematopoietic progenitor cells and MSCs, in the desired ratios or proportions, in order to obtain a cell population. The skilled person in the art will understand that said cell population may be enriched in any type of specific cells by conventional means, for example, by separating a specific type of cells by any suitable technique based on the use of binding pairs for the corresponding surface markers.

In a preferred embodiment the cell or cell population of the invention is isolated. The term "isolated" applied to a cell or a cell population refers to a cell or cell population, isolated from the human or animal body, which is substantially free of one or more cell populations that are associated with said cell or cell population *in vivo* or *in vitro.*

In a particular embodiment, the cell for use in the treatment of Parkinsonism is a hematopoietic stem cell. In another preferred embodiment, the cell for use in the treatment of Parkinsonism is a hematopoietic progenitor cell. In another preferred embodiment, the cell for use in the treatment of Parkinsonism is a mesenchymal stem cell.

The invention also relates to a cell population for use in the treatment of Parkinsonism, wherein the cell population comprises, consists essentially of or consists of any combination of a hematopoietic stem cell, a hematopoietic progenitor cell and a mesenchymal stem cell.

In a preferred embodiment, the cell population for use according to the invention comprises, consists essentially of, or consists of one cell selected from the group consisting of a hematopoietic stem cell, a hematopoietic progenitor cell and a mesenchymal stem cell. Therefore, in a preferred embodiment, the cell population for use according to the invention comprises, consists essentially of, or consists of a hematopoietic stem cell. In another preferred embodiment, the cell population for use according to the invention comprises, consists essentially of or consists of a hematopoietic progenitor cell. In another preferred embodiment, the cell population for use according to the invention comprises, consists essentially of or consists of a mesenchymal stem cell.

In another preferred embodiment, the cell population for use according to the invention comprises, consists essentially of, or consists of two cells selected from the group consisting of a hematopoietic stem cell, a hematopoietic progenitor cell, and a mesenchymal stem cell. Therefore, the cell population for use according to the invention comprises, consists essentially of, or consists of a hematopoietic stem cell and a hematopoietic progenitor cell; a hematopoietic stem cell and a mesenchymal stem cell; or a hematopoietic progenitor cell and a mesenchymal stem cell.

In a more preferred embodiment, the cell population comprises, consists essentially of, or consists of a hematopoietic stem cell and a hematopoietic progenitor cell. In another preferred embodiment, the cell population further comprises a mesenchymal stem cell.

In another preferred embodiment, the cell population for use according to the invention comprises, consists essentially of, or consists of a hematopoietic stem cell (HSC), a hematopoietic progenitor cell (HPC), and a mesenchymal stem cell (MSC).

The cell population comprising, consisting essentially of or consisting of a hematopoietic stem cell and a hematopoietic progenitor cell is usually named as HSPC. In a preferred embodiment the HSPCs are identified amongst others by presence of various antigenic markers on their surface. In this regards, the HSPC cell population in humans can be obtained either by purification on the basis of CD34 expression or on the basis of lineage depletion (lack of lineage markers) of CD2, CD3, CD11b, CD14, CD15, CD16, CD19, CD56, CD123 and CD235a markers, or by doing both methods.

Lineage depletion (lack of lineage markers) is obtained by using a standard cocktail of antibodies (CD2, CD3, CD11b, CD14, CD15, CD16, CD19, CD56, CD123 and CD235a markers) designed to remove mature hematopoietic cells from a sample.

"CD2", as used herein, relates to cluster of differentiation 2 and is a cell adhesion molecule. The complete protein sequence for human CD2 has the Uniprot accession number P06729 (March 16, 2016).

"CD3", as used herein, also known as cluster of differentiation 3, consists of a protein complex and is composed of four distinct chains. In mammals, the complex contains a CD3γ chain, a CD3δ chain, and two CD3ε chains. The complete protein sequence for human CD3δ has the Uniprot accession number P04234 (March 16, 2016).

"CD11b", as used herein, is one protein subunit that forms the heterodimeric integrin alpha-M beta-2 (αMβ2) molecule, also known as macrophage-1 antigen (Mac-1) or complement receptor 3 (CR3). The complete protein sequence for human CD11b has the Uniprot accession number P11215 (March 16, 2016).

"CD14" (cluster of differentiation 14) exists in two forms, one anchored to the membrane by a glycosylphosphatidylinositol tail (mCD14), the other a soluble form (sCD14). The complete protein sequence for human CD14 has the Uniprot accession number P08571 (March 16, 2016).

"CD15" (3-fucosyl-N-acetyl-lactosamine) is a carbohydrate adhesion molecule that can be expressed on glycoproteins, glycolipids and proteoglycans. The complete protein sequence for human CD15 has the Uniprot accession number P22083 (March 16, 2016).

"CD16" is a low affinity Fc receptor. The complete protein sequence for human CD16a has the Uniprot accession number P08637 (March 16, 2016).

"CD19" is a cell surface molecule that assembles with the antigen receptor of B lymphocytes in order to decrease the threshold for antigen receptor-dependent stimulation. The complete protein sequence for human CD19 has the Uniprot accession number P15391 (March 16, 2016).

"CD56", also known as Neural cell adhesion molecule (NCAM), is a homophilic binding glycoprotein expressed on the surface of neurons, glia, skeletal muscle and natural killer cells. The complete protein sequence for human CD56 has the Uniprot accession number P13591 (March 16, 2016).

"CD123", also known as interleukin-3 receptor, "IL3RA", Interleukin 3 receptor, alpha (low affinity) (IL3RA), is a molecule found on cells which helps transmit the signal of interleukin-3. The complete protein sequence for human CD123 has the Uniprot accession number P26951 (March 16, 2016). The term "IL3RA", as used herein, refers to Interleukin 3 receptor, alpha (low affinity) (IL3RA), also known as CD123 (Cluster of Differentiation 123).

"CD235a", Glycophorin A (MNS blood group), also known as GYPA, is a single-pass transmembrane glycoprotein. The complete protein sequence for human CD235a has the Uniprot accession number P02724 (March 16, 2016).

The term "CD34", as used herein, refers to a cluster of differentiation present on certain cells within the human body. It is a cell surface glycoprotein and functions as a cell-cell adhesion factor. It may also mediate the attachment of stem cells to bone marrow extracellular matrix or directly to stromal cells. Cells expressing CD34 (CD34+ cell) are normally found in the umbilical cord and bone marrow as hematopoietic cells, a subset of mesenchymal stem cells, endothelial hematopoietic progenitor cells, endothelial cells of blood vessels but not lymphatic cells. The complete protein sequence for human CD34 has the UniProt accession number P28906 (March 16, 2016).

In a preferred embodiment, the cell population comprising, consisting essentially of, or consisting of a hematopoietic stem cell and a hematopoietic progenitor cell, is from human origin, and more preferably is CD34+.

Murine HSPCs (cell population comprising hematopoietic stem and hematopoietic progenitor cells) can be obtained by the purification on the basis of lineage depletion (lack of lineage markers) of CD5, CD45R (B220), CD11b, Ly6G, Ly6B.2 (7/4), and Ter-119.

The term "CD5", as used herein, refers to a cluster of differentiation found on a subset of IgM-secreting B cells called B-1 cells. The complete protein sequence for murine CD5 has the UniProt accession number P13379 (March 16, 2016).

The term "CD45", as used herein, refers to a family consisting of multiple members that are all products of a single complex gene. This gene contains 34 exons, and three exons of the primary transcripts are alternatively spliced to generate up to eight different mature mRNAs and, after translation, eight different protein products. These three exons generate the RA, RB and RC isoforms. Various isoforms of CD45 exist: CD45RA, CD45RB, CD45RC, CD45RAB, CD45RAC, CD45RBC, CD45R0, CD45R (ABC). The complete protein sequence for murine CD45 has the UniProt accession number P06800 (April 13, 2016).

The term "Mac-1 (CD11b)", as used herein, refers to a Integrin alpha M (ITGAM) and it is one protein subunit that forms the heterodimeric integrin alpha-M beta-2 (αMβ2) molecule, also known as macrophage-1 antigen (Mac-1) or complement receptor 3 (CR3). ITGAM is also known as CR3A, and cluster of differentiation molecule 11B (CD11B). The complete protein sequence for murine Mac-1 has the UniProt accession number P05555 (March 16, 2016).

The term "Ly6G", as used herein, refers to Ly6G (Lymphocyte antigen 6 complex locus G6D). It is a 21-25 kDa glycosylphosphatidylinositol (GPI)-linked differentiation antigen that is expressed by myeloid-derived cells in a tightly developmentally-regulated manner in the bone marrow. The complete protein sequence for murine Ly6G has the UniProt accession number P35461 (February 17, 2016).

The term "Ly6B.2 (7/4)", as used herein, refers to a member of the Lymphocyte Antigen 6 (Ly6) superfamily and is located on chromosome space15 as the other members of the murine Ly6 family. The Ly6 family members are normally GPI-anchored, cysteine-rich cell surface molecules. Expression of Ly6B.2 antigen has been reported on neutrophils, bone marrow restricted progenitors, monocytes and is lost during the differentiation of monocytes into tissue resident macrophages or dendritic cell phenotype. The Gene ID of murine Ly6 has the accession number 23936 in the NCBI database (April 3, 2016).

The term "Ter-119" or the lymphocyte antigen 76 (Ly76), as used herein, refers to the Ter-119 antigen which is expressed on mature erythrocytes and erythroid precursor cells in adult blood, spleen, and bone marrow, and in the embryonic yolk sac and fetal liver. The complete protein sequence for murine Ly76 has the UniProt accession number P35461 (February 17, 2016).

The term "Hematopoietic stem cell" or "HSC", in plural "HSCs", as used herein, refers to a multipotent stem cell that gives rise to all the blood cell types from the myeloid (monocytes and macrophages, neutrophils, basophils, eosinophils, erythrocytes, megakaryocytes/platelets, dendritic cells), and lymphoid lineages (T-cells, B-cells, NK-cells). HSCs are a heterogeneous population. Human HSC may be collected by way of illustrative non-limitative example from bone marrow, peripheral blood or umbilical cord blood.

In a preferred embodiment the HSC is a mammalian cell, preferably a human cell.

In a preferred embodiment, the human HSC is CD34+/CD38-/CD90+/CD45RA-. In another preferred embodiment, the HSC is CD34-/CD38-.

The term "CD38", as used herein, refers to cluster of differentiation 38, also known as cyclic ADP ribose hydrolase. It is a glycoprotein found on the surface of many immune cells (white blood cells), including CD4+, CD8+, B and natural killer cells. CD38 also functions in cell adhesion, signal transduction and calcium signalling. CD38 is a type II transmembrane protein that functions as a signalling molecule and mediates the adhesion between lymphocytes and endothelial cells. It also functions enzymatically in the formation and hydrolyzation of the second messenger cyclic ADP ribose. In the hematopoietic system, CD38 is most highly expressed on plasma cells. The complete protein sequence for human CD38 has the UniProt accession number P28907 (March 16, 2016).

The term "CD90" or Thy-1, as used herein, refers to cluster of differentiation 90, a 25-37 kDa heavily N-glycosylated, glycophosphatidylinositol (GPI) anchored conserved cell surface protein with a single V-like immunoglobulin domain, originally discovered as a thymocyte antigen. The immunoglobulin domain is a type of protein domain that consists of a 2-layer sandwich of between 7 and 9 antiparallel β-strands arranged in two β-sheets with a Greek key topology. The complete protein sequence for human CD90 has the UniProt accession number P04216 (March 16, 2016)

The term "CD45", as used herein, refers to a family consisting of multiple members that are all products of a single complex gene. This gene contains 34 exons and three exons of the primary transcripts are alternatively spliced to generate up to eight different mature mRNAs and, after translation, eight different protein products. These three exons generate the RA, RB and RC isoforms. Various isoforms of CD45 exist: CD45RA, CD45RB, CD45RC, CD45RAB, CD45RAC, CD45RBC, CD45R0, CD45R (ABC). The complete protein sequence for human CD45 has the UniProt accession number P08575 (March 16, 2016).

The term "lin" refers to lineage markers, a standard cocktail of antibodies designed to remove mature hematopoietic cells from a sample.

The murine hematopoietic stem cell (HSC) is characterized by the KSL markers; c-kit+ (CD117+), Sca-1+, and lineage - (lin-). Currently, the hematopoietic stem cell is characterized by CD150+/CD48-/CD34^{low}/ KSL.

The term "c-Kit" refers to a Mast/stem cell growth factor receptor (SCFR), also known as proto-oncogene c-Kit or tyrosine-protein kinase Kit or CD117. It is a protein that in humans is encoded by the Kit gene. CD117 is a receptor tyrosine kinase type III, which binds to stem cell factor, also known as "steel factor" or "c-Kit ligand". The complete protein sequence for murine c-Kit has the UniProt accession number P05532 (March 16, 2016).

The term "SCA-1" refers to ataxin 1, which function is unknown. The complete protein sequence for murine SCA-1 has the UniProt accession number P54254 (April 13, 2016).

The term "SLAMF1", as used herein, refers to signalling lymphocytic activation molecule. It is a protein that in humans is encoded by the SLAMF1 gene. SLAMF1 has also recently been designated CD150 (cluster of differentiation 150). The complete protein sequence for murine SLAMF1 has the UniProt accession number Q9QUM4 (April 13, 2016).

CD48 antigen (Cluster of Differentiation 48), also known as B-lymphocyte activation marker (BLAST-1) or signaling lymphocytic activation molecule 2 (SLAMF2), is a protein that in humans is encoded by the CD48 gene. CD48 is a member of the CD2 subfamily of the immunoglobulin superfamily (IgSF), which includes SLAM (signaling lymphocyte activation molecules) proteins. The complete protein sequence for murine SLAMF2 has the UniProt accession number P18181 (March 16, 2016).

A "hematopoietic progenitor cell" or "HPC", refers to a cell that is derived from a stem cell by differentiation and is capable of further differentiation to more mature cell types. Hematopoietic progenitor cells typically have more restricted proliferation capacity as compared to stem cells. The hematopoietic progenitor cell may be derived from a HSC by differentiation during the progression from HSCs to differentiated functional cells.

In a preferred embodiment the hematopoietic progenitor cell is a mammalian cell, preferably a human cell. The markers CD34+/CD38-/CD90-/CD45RA- characterize the human hematopoietic progenitor cell.

In another particular embodiment the hematopoietic progenitor cell is a Common Myeloid Progenitor (CMP), i.e., a colony-forming unit that generates myeloid cells characterized by CD34+/CD38+/IL3RA^{low}/CD45RA-.

In another particular embodiment the hematopoietic progenitor cell is a Granulocyte-Macrophage Progenitor (GMP), the precursor for monoblasts and myeloblasts characterized by CD34+/CD38+/IL3RA-/CD45RA-.

In another particular embodiment, the hematopoietic progenitor cell is a Megakaryocyte-Erythroid Progenitor (MEP) characterized by CD34+/CD38+/IL3RA+/CD45RA+.

The term "mesenchymal stem cell " or "MSC", in plural "MSCs", as used herein, refers to a multipotent stromal cell that can differentiate into a variety of cell types, including: osteoblasts (bone cells), chondrocytes (cartilage cells), and adipocytes (fat cells). Markers expressed by human and mouse mesenchymal stem cells include CD105 (SH2), CD73 (SH3/4), CD44, CD90 (Thy-1), CD71 and STRO-1 as well as the adhesion molecules CD106, CD166, and CD29. Among negative markers for MSCs (not expressed) are hematopoietic markers CD45, CD34, CD14, and the costimulatory molecules CD80, CD86 and CD40 as well as the adhesion molecule CD31.

The term "CD105", as used herein, refers to endoglin, a type I membrane glycoprotein located on cell surfaces that is part of the TGF beta receptor complex. The complete protein sequence for human CD105 has the UniProt accession number P17813 (March 16, 2016).

The term "CD73", as used herein, refers to 5'-nucleotidase (5'-NT), also known as ecto-5'-nucleotidase or CD73 (Cluster of Differentiation 73). It is an enzyme that in humans is encoded by the NT5E gene. The complete protein sequence for human CD73 has the UniProt accession number P21589 (March 16, 2016).

The term "CD44" refers to CD44 antigen, also called HCAM (homing cell adhesion molecule), Pgp-1 (phagocytic glycoprotein-1), Hermes antigen, lymphocyte homing receptor, ECM-III or HUTCH-1. It is a cell-surface glycoprotein involved in cell-cell interactions, cell adhesion and migration. The complete protein sequence for human CD44 has the UniProt accession number P16070 (March 16, 2016).

The term "CD71 ", as used herein, refers to Transferrin receptor protein 1 (TfR1) also known as cluster of differentiation 71. It is a protein that is required for iron delivery from transferrin to cells. The complete protein sequence for human CD71 has the UniProt accession number P02786 (March 16, 2016).

The term "STRO-1", as used herein, refers to a cell surface protein expressed by bone marrow stromal cells and erythroid precursors. The STRO-1 may be identified using an antibody STRO-1 such as sc-47733 from Santa Cruz Biotechnology.

The term "CD106" refers to a Vascular cell adhesion protein 1 also known as vascular cell adhesion molecule 1 (VCAM-1) or cluster of differentiation 106 (CD106). It is a protein that in humans is encoded by the VCAM1 gene and functions as a cell adhesion molecule. The complete protein sequence for human CD106 has the UniProt accession number P19320 (March 16, 2016).

The term "CD166", as used herein, refers to a 100-105 kDa type I transmembrane glycoprotein that is a member of the immunoglobulin superfamily of proteins. The complete protein sequence for human CD166 has the UniProt accession number Q13740 (March 16, 2016).

The term "CD29", as used herein, refers to an integrin beta-1. It is an integrin unit associated with very late antigen receptors. The complete protein sequence for human CD29 has the UniProt accession number P05556 (March 16, 2016).

The term "CD14", as used herein, refers to cluster of differentiation 14 which is a component of the innate immune system. The complete protein sequence for human CD14 has the UniProt accession number P08571 (March 16, 2016).

The term "CD80", as used herein, refers to cluster of differentiation 80 (also CD80 and B7-1). It is a protein found on activated B cells and monocytes that provides a costimulatory signal necessary for T cell activation and survival. The complete protein sequence for human CD80 has the UniProt accession number P33681 (March 16, 2016).

The term "CD86", as used herein, refers to cluster of differentiation 86 (also known as CD86 and B7-2). It is a protein expressed on antigen-presenting cells that provides costimulatory signals necessary for T cell activation and survival. The complete protein sequence for human CD86 has the UniProt accession number P42081 (March 16, 2016).

The term "CD40", as used herein, refers to a costimulatory protein found on antigen presenting cells and is required for their activation. The complete protein sequence for human CD40 has the UniProt accession number P25942 (March 16, 2016).

The term "CD31", as used herein, refers to a Platelet endothelial cell adhesion molecule (PECAM-1), also known as cluster of differentiation 31 (CD31). It is a protein that plays a key role in removing aged neutrophils from the body. The complete protein sequence for human CD31 has the UniProt accession number P16284 (March 16, 2016).

The presence/absence of a marker in a cell can be determined, for example, by means of flow cytometry using conventional methods and apparatuses. For instance, a BD LSR II Flow Cytometer (BD Biosciences Corp., Franklin Lakes, NJ, US) with commercially available antibodies and following protocols known in the art may be employed. Thus, cells emitting a signal for a specific cell surface marker more intense than the background noise can be selected. The background signal is defined as the signal intensity given by a non-specific antibody of the same isotype as the specific antibody used to detect each surface marker in the conventional FACS analysis. In order for a marker to be considered positive, the observed specific signal must be 10%, 20%, preferably, 30%, 40%, 50%, 60%, 70%, 80%, 90%, 500%, 1000%, 5000%, 10000% or above more intense than the background signal using conventional methods and apparatuses (e.g. by using a FACSCalibur flow cytometer (BD Biosciences Corp., Franklin Lakes, NJ, US) and commercially available antibodies). Otherwise the cell is considered negative for said marker.

The low or high level of a marker in a cell may be determined as previously described. A skilled person knows the reference value of a marker in order to determine if the level is low or high. Low level of a marker relates to a level lower than the reference value. The level of a marker is considered to be lower than a reference value when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more lower than its reference value. High level of a marker relates to a level higher than the reference value. The level of a marker is considered to be higher than a reference value when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than its reference value.

In a particular embodiment, the cell for use in the treatment of Parkinsonism is a HSC.

In another particular embodiment, the cell for use in the treatment of Parkinsonism is a hematopoietic progenitor cell.

The cells or cell populations of the invention are used for the treatment of Parkinsonism.

The term "treatment", as used herein, refers to both therapeutic measures and prophylactic or preventive measures, wherein the object is to prevent or slow down (lessen) an undesired physiological change or disorder, such as parkinsonism. Beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

The term "subject" or "patient" is meant any subject, particularly a mammalian subject, for whom therapy is desired. Mammalian subjects include humans, domestic animals, farm animals, and zoo, sports, or pet animals such as dogs, cats, guinea pigs, rabbits, rats, mice, horses, cattle, cows, and so on. In a preferred embodiment of the invention, the subject is a mammal. In a more preferred embodiment of the invention, the subject is a human.

The term "Parkinsonism", as used herein, relates to a clinical syndrome manifested by a combination of motor symptoms such as tremor, bradykinesia, rigidity, postural instability, flexed posture and freezing. Classifications of Parkinsonism are Parkinson's Disease, secondary Parkinsonism, hereditary Parkinsonism and Parkinsonism Plus Syndromes (multisystem degenerations).

In a first aspect, the invention relates to the treatment of Parkinsonism that results from progressive dopaminergic neuron degeneration.

Parkinsonism that results from progressive dopaminergic neuron degeneration includes Parkinson's Disease (sporadic and hereditary), but may also include cases of secondary Parkinsonism caused by, for example, toxic chemicals, drugs, viruses, bacteria, heavy metals, trauma, etc. and Parkinsonism Plus Syndromes.

In a more preferred embodiment, Parkinsonism is Parkinson's disease.

Parkinson's disease is the most common neurodegenerative cause of Parkinsonism, a clinical syndrome characterized by lesions in the basal ganglia, predominantly in the substantia nigra.

In a preferred embodiment of the medical use of the invention, the cell or the cell population for use according to the invention is transplanted, injected, infused or administered into the brain of a subject in need of treatment, preferably in the midbrain, more preferably, above the substantia nigra pars compacta (SNpc).

In a preferred embodiment, the cell or the cell population for use according to the invention is transplanted in the midbrain.

"Transplanted", as used herein, relates to a procedure that infuses cells to replace damaged cells. Survival of transplanted cells in a living subject may be determined through the use of a variety of scanning techniques, e.g., computerized axial tomography (CAT or CT) scan, magnetic resonance imaging (MRI) or positron emission tomography (PET) scans. Alternatively, determination of transplant survival may also be done post mortem by removing the tissue and examining it visually or through a microscope. Examining restoration of the brain function that was damaged or diseased can assess functional integration of transplanted cells into brain tissue of a subject. For example, effectiveness in the treatment of Parkinson may be determined by recovery of dopamine release. The transplantation of the cells may be performed using any method known in the art, such as the method disclosed in the experimental part of the present application.

"Brain", as used herein, relates to the mass of nerve tissue in the anterior end of an organism.

"Midbrain" or "mesencephalon", as used herein, relates to a portion of the brain composed of the tectum and tegmentum, and it includes the cerebral aqueduct and cerebral peduncles.

In a more preferred embodiment, the cell or the cell population for use according to the invention is transplanted above the substantia nigra pars compacta (SNpc)

"Substantia nigra pars compacta (SNpc)", as used herein, relates to a regional part of substantia nigra consisting of a densely packed region of cells, more or less dorsal to the pars reticulata, but extending into the pars reticulata in some species. The dominant neurotransmitter used by pars compacta neurons is dopamine. Pars compacta neurons are pigmented in many species.

Above or dorsal to the substantia nigra pars compacta (SNpc) in the midbrain area relates to an area known by a skilled person.

In another preferred embodiment, the cell or cell population for use according to the invention is transplanted, injected, infused or administered unilaterally.

"Unilaterally", as used herein, relates to the transplantation/injection/infusion or administration of a cell or cell population of the invention in only one side of the brain.

In another preferred embodiment, the cell or cell population for use according to the invention is transplanted, injected, infused or administered bilaterally.

"Bilaterally", as used herein, relates to the transplantation/injection/infusion or administration of a cell or cell population of the invention in both sides of the brain.

For use within the teachings of the present invention the cell or cell population may be from the same subject, i.e., autologous, in order to minimize the risk of eventual rejections or undesired side reactions. Nevertheless, the invention also contemplates the use of allogeneic cells, i.e., cells from other subject of the same species as that of the recipient subject in which case the use of systemic or local immunosuppressive agents may be recommended.

In another embodiment, the cell or the cell population for use according to the invention is transplanted into a region of the brain having the Wnt/β-catenin signaling pathway activated or in a region of the brain wherein the Wnt/β-catenin signaling pathway is activated after transplantation.

A skilled person knows techniques to determine if an area of the brain shows Wnt/β-catenin signaling pathway activated. By way of illustrative non-limitative example, the presence of Wnt may be detected in the cerebrospinal fluid as indicative that the Wnt/β-catenin signaling pathway is activated.

This means that the subject to be treated shows the Wnt/β-catenin signaling pathway activated in a region of the brain, preferably in the midbrain, more preferably, in the substantia nigra pars compacta (SNpc). Said activation may be an endogenous activation, for example as a consequence of a damage, lesion or injury in said region of the subject. Alternatively, Wnt/β-catenin signaling pathway in said region may be exogenously activated by a Wnt/β-catenin signaling pathway activator, or by an inhibitor of a Wnt/β-catenin signaling pathway repressor. Suitable Wnt/β-catenin signaling pathway activator, or inhibitor of a Wnt/β-catenin signaling pathway repressor are disclosed below.

In another embodiment of the present invention, the Wnt/β-catenin signalling pathway of the cell or cell population for use according to the invention is activated. The Wnt/β-catenin signalling pathway may be activated in a cell by treating said cell with a Wnt/β-catenin signalling pathway activator o with an inhibitor of a Wnt/β-catenin signalling pathway repressor or by overexpressing a Wnt/β-catenin signalling pathway activator or by overexpressing an inhibitor of the Wnt/β-catenin signalling pathway repressor in said cell.

In another embodiment, the use for the treatment of Parkinsonism, further comprises the previous, simultaneous, sequential or separate administration, injection or infusion to the subject in need of treatment of a Wnt/β-catenin signalling pathway activator or an inhibitor of a Wnt/β-catenin signalling pathway repressor.

In another preferred embodiment, the use for the treatment of Parkinsonism, further comprises the previous, simultaneous, sequential or separate administration, injection or infusion to the subject in need of treatment of a neurotrophic factor.

"Neurotrophic factor", as used herein, relates to proteins that are responsible for the growth and survival of developing neurons and the maintenance of mature neurons. By way of illustrative non-limitative example, a neurotrophic factor may be BDNF or GDNF.

"BDNF", as used herein, relates to a member of the neurotrophin family of growth factors, which are related to the canonical Nerve Growth Factor. The complete sequence of BDNF in humans has the Uniprot accession number P23560 (March 16, 2016).

"GDNF", as used herein, relates to a small protein that potently promotes the survival of many types of neurons. The complete sequence of GDNF in humans has the Uniprot accession number P39905 (March 16, 2016).

"Previous", as used herein, relates to the administration of a Wnt/β-catenin signalling pathway activator or an inhibitor of a Wnt/β-catenin signalling pathway repressor at a time earlier than the administration of the cell or cell population of the invention. The delay between the two administrations may be seconds, minutes, hours or days.

"Simultaneous" administration, as used herein, means at the same time, while"Sequential" administration preferably means administration of the cell or cell population of the invention at one time point, and a Wnt/β-catenin signalling pathway activator or an inhibitor of a Wnt/β-catenin signalling pathway repressor at a different time point, in a staggered manner.

"Separate" administration preferably means administration of the cell or cell population of the invention and a Wnt/β-catenin signalling pathway activator or an inhibitor of a Wnt/β-catenin signalling pathway repressor at a different time point, independently of each other at different time points.

When administered sequentially or separately, the cell or cell population of the invention and a Wnt/β-catenin signalling pathway activator or an inhibitor of a Wnt/β-catenin signalling pathway repressor, may be administered in any order. In one particular embodiment, the cell or cell population of the invention, is administered first. In another particular embodiment, a Wnt/β-catenin signalling pathway activator or an inhibitor of a Wnt/β-catenin signalling pathway repressor is administered first. The particular period of delay between the administrations of the components can extend to days, hours, minutes or seconds.

In another embodiment, the cell or cell population of the invention are pretreated with a Wnt/β-catenin signalling pathway activator, and/or with an inhibitor of a Wnt/β-catenin signalling pathway repressor, and/or said cells overexpress a Wnt/β-catenin signalling pathway activator, and/or said cells overexpress an inhibitor of a Wnt/ β-catenin signalling pathway repressor.

The expression "Wnt/β-catenin signalling pathway" refers to a network of proteins that play a variety of important roles in embryonic development, cell differentiation, pluripotency and self-renewal maintenance, somatic cell reprogramming, and cell polarity generation. Unless otherwise indicated, it refers to the canonical Wnt pathway and includes a series of events that occur when Wnt proteins bind to cell-surface receptors of the Frizzled family, causing the receptors to activate Dishevelled family proteins and ultimately resulting in a change in the amount of β-catenin that reaches the nucleus. Dishevelled (DSH) is a key component of a membrane-associated Wnt receptor complex, which, when activated by Wnt binding, inhibits a second complex of proteins that includes axin, glycogen synthase kinase 3 (GSK-3), and the protein adenomatous polyposis coli (APC). The axin/GSK-3/APC complex normally promotes the proteolytic degradation of the β-catenin intracellular signalling molecule. After this β-catenin destruction complex is inhibited, a pool of cytoplasmic β-catenin stabilizes, and some β-catenin, is able to enter the nucleus and interact with TCF/LEF family transcription factors to promote specific gene expression. Several protein kinases and protein phosphatases have been associated with the ability of the cell surface Wnt-activated Wnt receptor complex to bind axin and disassemble the axin/GSK3 complex. Phosphorylation of the cytoplasmic domain of LRP by CK1 and GSK3 can regulate axin binding to LRP. The protein kinase activity of GSK3 appears to be important for both the formation of the membrane-associated Wnt/FRZ/LRP/DSH/Axin complex and the function of the Axin/APC/GSK3/β-catenin complex. Phosphorylation of β-catenin by GSK3 leads to the destruction of β-catenin.

A "Wnt/β-catenin signalling pathway activator", as used herein, refers to a molecule capable of activating the Wnt/β-catenin signalling pathway. In general, the Wnt/β-catenin signalling pathway is activated when the target genes are transcribed; by way of illustration, activation of the Wnt/β-catenin signalling pathway may be confirmed by analyzing the expression of the target genes, e.g., Axin2, Myc, Cyclin D1, TCF-1 by RT-PCR, or by detection of β-catenin translocation in the nuclei of the cells by, e.g., immunostaining, or by detecting the phosphorylation of Dishevelled or the phosphorylation of the LRP tail, etc. Wnt/β-catenin signalling pathway activators may act on membrane receptors of Wnt signalling proteins and on the proteins that comprise the signalling cascade. Illustrative, non-limiting examples of Wnt/β-catenin signalling pathway activators include peptides or proteins as well as chemical compounds other than peptides or proteins (i.e., non-peptide drugs), such as:
- peptides or proteins, for example, Wnt protein isoforms such as Wnt1, Wnt2, Wnt2b/13, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11 or Wnt16; β-catenin; a spondin, such as a R-spondin, etc.; or functional variants thereof, e.g., peptides or proteins that have an amino acid sequence that is at least 40%, typically at least 50%, advantageously at least 60%, preferably at least 70%, more preferably at least 80%, still more preferably at least 90% identical to the amino acid sequences of the previously mentioned peptides or proteins and that maintain the ability to activate the Wnt/β-catenin signalling pathway; or
- non-peptide compounds, for example, 2-(4-acetylphenylazo)-2-(3,3-dimethyl-3,4-dihydro-2H-isoquinolin-1-ylidene)-acetamide (IQ1), (2S)-2-[2-(indan-5-yloxy)-9-(1,1'-biphenyl-4-yl)methyl)-9H-purin-6-ylamino]-3-phenyl-propan-1-ol (QS11), deoxycholic acid (DCA), 2-amino-4-[3,4-(methylenedioxy)benzylamino]-6-(3-methoxyphenyl)pyrimidine, or an (hetero)arylpyrimidine as those disclosed by Gilbert et al., in Bioorganic & Medicinal Chemistry Letters, 20(1):366-370 (2010).

Examples of Wnt protein isoforms, which belong to the Wnt secreted proteins family and act as activators of the Wnt/β-catenin signalling pathway, include the following or orthologues thereof (Swiss-prot references):
Homo sapiens: Wnt1: P04628; Wnt2: P09544; Wnt2b/13: Q93097; Wnt3: P56703; Wnt3a: P56704; Wnt4: P56705; Wnt5a: P41221; Wnt5b: Q9H1J7; Wnt6: Q9Y6F9; Wnt7a: 000755; Wnt7b: P56706; Wnt8a: Q9H1J5; Wnt9a: 014904; Wnt9b: 014905; Wnt10a: Q9GZT5; Wnt10b: 000744; Wnt11: 096014; Wnt16: Q9UBV4;
Mus musculus: Wnt1: P04426; Wnt2: P21552.1; Wnt2b/13: 070283.2; Wnt3: P17553; Wnt3a: P27467; Wnt4: P22724; Wnt5a: P22725; Wnt5b: P22726; Wnt6: P22727.1; Wnt8a: Q64527; Wnt9a: Q8R5M2; Wnt9b: 035468.2; Wnt10a: P70701; Wnt10b: P48614; Wnt11: P48615; Wnt16: Q9QYSI.I;
as well as a functional isoform, variant or fragment thereof, i.e., an isoform, variant or fragment thereof having the ability to activate the Wnt/β-catenin signalling pathway.

Examples of β-catenin include the following or orthologues thereof (Swiss-prot references):
Homo sapiens: P35222;
Mus musculus: Q02248;
as well as a functional isoform, variant or fragment thereof, i.e., an isoform, variant or fragment thereof having the ability to activate the Wnt/β-catenin signalling pathway.

The R-Spondins (RSpo) are 4 secreted agonists of the canonical Wnt/β-catenin signalling pathway. Also known as cysteine-rich and single thrombospondin domain containing proteins (Cristins), R-Spondins share around 40% amino acid identity (Lowther, W. et al. (2005) J. Virol. 79:10093; Kim, K.-A. et al. (2006) Cell Cycle 5:23). All the R-Spondins contain two adjacent cysteine-rich furin-like domains followed by a thrombospondin (TSP-1) motif and a region rich in basic residues. Only the furin-like domains are needed for β-catenin stabilization (Kim, K.-A. et al. (2006) Cell Cycle 5:23; Kazanskaya, O. et al. (2004) Dev. Cell 7:525). Injection of recombinant R-Spondin 1 in mice causes activation of β-catenin and proliferation of intestinal crypt epithelial cells, and ameliorates experimental colitis (Kim, K.-A. et al. (2005) Science 309:1256; Zhao, J. et al. (2007) Gastroenterology 132:1331). R-Spondin 1 (RSPO1) appears to regulate Wnt/β-catenin by competing with the Wnt antagonist DKK-1 for binding to the Wnt co-receptor, Kremen. This competition reduces internalization of DKK-1/LRP-6/Kremen complexes (Binnerts, M.E. et al. (2007) Proc. Natl. Acad. Sci. USA 104:147007). Illustrative, non-limitative, examples of R-Spondins which act as activators of the Wnt/β-catenin signalling pathway, include the following or orthologues thereof (Swiss-prot references):
Homo sapiens: R-spondin-1: Q2MKA7; R-spondin-2: Q6UXX9; R-spondin-3: Q9BXY4; R-spondin-4: Q2IOM5, or a functional isoform, variant or fragment thereof, i.e., an isoform, variant or fragment thereof having the ability to activate the Wnt/β-catenin signalling pathway, for example, an isoform, variant or fragment thereof that maintain their functional domains.

Illustrative, non-limitative, examples of (hetero)arylpyrimidines include the compounds of formula (I)-(IV) below.

In a particular embodiment, the (hetero)arylpyrimidine is an (hetero)aryl-pyrimidine agonist of the Wnt/β-catenin signalling pathway of formula (I), (II), (III) or (IV) [Table I].

**Table I**

| **Illustrative examples of (hetero)arylpyrimidines agonists of the Wnt/β-catenin signalling pathway** | | |
|---|---|---|
| **Compound of formula** | **Formula** | **Definitions** |
| (I) | 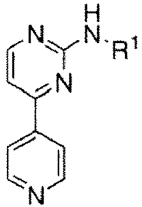 | R¹ is N-(3-1*H*-imidazol-1-yl)propane), N-(2-pyridin-4-yl)ethane), N-(2-pyridin-3-yl)ethane), N-(3-(3,5-dimethyl-1*H*-pyrazol-1-yl)propyl), N-(2-(1*H*-indol-3-yl)ethane), or N-(S)-3-(1*H*-indol-3-yl)-2-propan-1-ol amine |
| (II) | 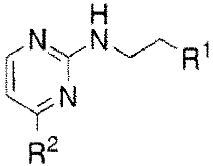 | R¹ is CH₂-1*H*-imidazole, 4-pyridine, 3-(1*H*-indole), 3-(2-methyl-1*H*-indol-5-ol), or 4-(1*H-*imidazole); and |
| | | R² is 4-(pyridin-4-yl), 4-(pyridin-3-yl), 4-(3-nitrophenyl), 2-(benzo[b]thiophene) or 2-(naphthyl) |
| (III) | 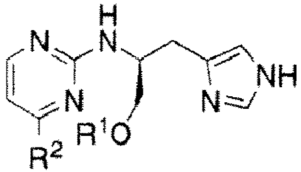 | R¹ is H, ethyl, methylenecyclohexyl, 2-fluoro-3-(trifluoromethyl)benzyl or prop-2-ynyl; and |
| | | R² is 2-(benzo[b]thiophene) or 2-(naphthyl) |
| (IV) | 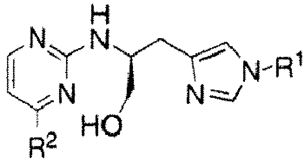 | R¹ is 3,5-difluorobenzyl, prop-2-ynyl, 2-acetamide or 3-propanitrile; and |
| | | R² is 2-(benzo[b]thiophene) or 2-(naphthyl) |

An "inhibitor of a Wnt/β-catenin signalling pathway repressor", as used herein, refers to a molecule capable of activating the Wnt/β-catenin signalling pathway by inhibiting or blocking a Wnt/β-catenin signalling pathway repressor, i.e., a compound which represses, blocks or silences the activation of the Wnt/β-catenin signalling pathway. Illustrative, non-limitative, examples of Wnt/β-catenin signalling pathway repressors include glycogen synthase kinase 3 (GSK-3), secreted frizzled-related protein 1 (SFRP1), and the like.

Illustrative, non-limitative, examples of inhibitors of SFRP1 include 5-(phenylsulfonyl)-N-(4-piperidinyl)-2-(trifluoromethyl)benzenesulfonamide (WAY-316606).

Illustrative, non-limitative, examples of inhibitors of GSK-3 include: lithium salts (e.g., lithium chloride), 6-bromoindirubin-3'-oxime (BIO), 6-bromoindirubin-3'-acetoxime (BIO-acetoxime), 6-{2-[4-(2,4-dichloro-phenyl)-5-(4-methyl-1H-imidazol-2-yl)-pyrimidin-2-ylamino]-ethyl-amino}-nicotino-nitrile (CHIR99021), N-[(4-methoxyphenyl)methyl]-N'-(5-nitro-2-thiazolyl)urea (AR-A014418), 3-(2,4-dichlorophenyl)-4-(1-methyl-1H-indol-3-yl)-1H-pyrrole-2,5-dione (SB-216763), 5-benzylamino-3-oxo-2,3-dihydro-1,2,4-thiadiazole (TDZD-20), 3-[(3-chloro-4-hydroxyphenyl)amino]-4-(2-nitro-phenyl)-1 H-pyrrole-2,5-dione (SB415286), 5-Ethyl-7,8-dimethoxy-1H-pyrrolo[3,4-c]isoquinoline-1,3(2H)-dione (3F8), N-[(4-Methoxyphenyl)methyl]-N'-(5-nitro-2-thiazolyl)urea (AR-A 014418), 6-[[2-[[4-(2,4-Dichlorophenyl)-5-(5-methyl-1 H-imidazol-2-yl)-2-pyrimidinyl]amino]ethyl]amino]-3-pyridinecarbonitrile (CHIR 99021), 6-[[2-[[4-(2,4-Dichlorophenyl)-5-(5-methyl-1 H-imidazol-2-yl)-2-pyrimidinyl]amino]ethyl]amino]-3-pyridinecarbonitrile trihydrochloride (CHIR 99021 trihydrochloride), 3-[[6-(3-Aminophenyl)-7H-pyrrolo[2,3-d]pyrimidin-4-yl]oxyphenol ditrifluoroacetate (TWS 119), 3-[5-[4-(2-Hydroxy-2-methyl-1-oxopropyl)-1-piperazinyl]-2-(trifluoromethyl)phenyl]-4-(1H-indol-3-yl)-1H-pyrrole-2,5-dione (TCS 21311), 2-Methyl-5-[3-[4-(methylsulfinyl)phenyl]-5-benzofuranyl]-1,3,4-oxadiazole (TCS 2002) etc., or functional analogs or derivatives thereof, i.e., compounds which contain functional groups which render the compound of interest when administered to a subject.

Further examples of GSK-3 inhibitors are known to those skilled in the art. Examples are described in, for example, WO 99/65897 and WO 03/074072 and references cited therein. For example, various GSK-3 inhibitor compounds are disclosed in US 2005/0054663, US 2002/0156087, WO 02/20495 and WO 99/65897 (pyrimidine and pyridine based compounds); US 2003/0008866, US 2001/0044436 and WO01/44246 (bicyclic based compounds); US 2001/0034051 (pyrazine based compounds); and WO 98/36528 (purine based compounds). Further GSK-3 inhibitor compounds include those disclosed in WO 02/22598 (quinolinone based compounds), US 2004/0077707 (pyrrole based compounds); US 2004/0138273 (carbocyclic compounds); US 2005/0004152 (thiazole compounds); and US 2004/0034037 (heteroaryl compounds). Further GSK-3 inhibitor compounds include macrocyclic maleimide selective GSK-3 β inhibitors developed by Johnson & Johnson and described in, for example, Kuo et al. (2003) J Med Chem 46(19):4021-31, a particular example being 10,11,13,14,16,17,19,20,22,23-Decahydro-9,4:24,29-dimetho-1H -dipyrido (2,3-n:3',2'-t) pyrrolo (3,4-q)-(1,4,7,10,13,22) tetraoxadiazacyclotetracosine-1,3 (2H)-dione. Further, substituted aminopyrimidine derivatives CHIR 98014 (6-pyridinediamine, N6-[2-[[4-(2,4-dichlorophenyl)-5-(1H-imidazol-1-yl)-2-pyrimidinyl]amino]ethyl]-3-nitro-) and CHIR 99021 (6-{2-[4-(2,4-dichloro-phenyl)-5-(4-methyl-1H-imidazol-2-yl)-pyrimidin-2-ylamino]-ethylamino}-nicotinonitrile) inhibit human GSK-3 potently. Also, a number of other GSK-3 inhibitors which may be useful in the present invention are commercially available from Calbiochem®, for example: 5-methyl-1H-pyrazol-3-yl)-(2-phenylquinazolin-4-yl)amine, 4-benzyl-2-methyl-1,2,4-thiadiazolidine-3,5-dione (TDZD8), 2-thio(3-iodobenzyl)-5-(1-pyridyl)-[1,3,4]-oxadiazole, 3-(1-(3-hydroxy-propyl)-1H-pyrrolo[2,3-b]pyridin-3-yl]-4-pyrazin-2-yl-pyrrole-2,5-dione, etc. Included within the scope of the invention are the functional analogs or derivatives of the above mentioned compounds.

For a review of compounds capable of activating the Wnt/β-catenin signalling pathway see Chen et al, Am J Physiol Gastrointest Liver Physiol. 299:G293-300, 2010; Barker et al., Nat Rev Drug Discov. 5:997-1014, 2006 and Meijer et al, Trends Pharmacol Sci. 25: 471-480, 2004.

In a particular embodiment, the compound used for activating Wnt/β-catenin signalling pathway is selected from the group consisting of a Wnt isoform, β-catenin, a R-spondin, or functional variants or fragments thereof, IQ1, QS11, DCA, 2-amino-4-[3,4-(methylenedioxy)-benzylamino]-6-(3-methoxyphenyl)pyrimidine, an (hetero)arylpyrimidine such as, for example, an (hetero)arylpyrimidine of formula (I), (II), (III) or (IV) [Table I], a GSK-3 inhibitor, a SFRP1 inhibitor, and any combinations thereof. In a particular embodiment, said Wnt protein isoform is selected from the group consisting of Wnt1, Wnt2, Wnt2b/13, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, Wnt16, and combinations thereof, or functional variants or fragments thereof. In another particular embodiment, said Wnt/β-catenin signalling pathway activator is β-catenin or a functional variant or fragment thereof. In another particular embodiment, said Wnt/β-catenin signalling pathway activator is a R-spondin such as R-spondin-1, R-spondin-2, R-spondin-3, R-spondin-4, or a functional isoform, variant or fragment thereof.

In another particular embodiment, the SFRP1 inhibitor is WAY-316606. In another particular embodiment, the GSK-3 inhibitor is selected from the group consisting of a lithium salt, preferably, lithium chloride, BIO, BIO-acetoxime, CHIR99021, AR-A014418, SB-216763, TDZD-20, SB415286, and any combination thereof. In another preferred embodiment, the inhibitor of a Wnt/β-catenin signaling pathway repressor is 2-amino-4-[3,4-(methylenedioxy)benzyl-amino]-6-(3-methoxyphenyl)pyrimidine

In a preferred embodiment, the Wnt/β-catenin signalling pathway activator is selected from the group consisting of Wnt3a, β-catenin, R-spondin-1, and a combination thereof. In another preferred embodiment, the inhibitor of the Wnt β-catenin signalling pathway repressor is selected from the group consisting of BIO, CHIR99021, and a combination thereof.

According to the invention, in order to activate the Wnt/β-catenin signalling pathway the cell or cell population of interest is contacted, e.g., cultured or incubated, with a Wnt/β-catenin signalling pathway activator or with an inhibitor of a Wnt/β-catenin signalling pathway repressor. The amount of said Wnt/β-catenin signalling pathway activator or inhibitor of a Wnt/β-catenin signalling pathway repressor may vary within a range; nevertheless, preferably, the compound will be added in a suitable amount, i.e., in an amount which allows to obtain a specific amount of β-catenin accumulated in the nucleus of the cells. Said amount can be determined by the skilled person in the art by conventional assays, for example, by contacting the cell with a Wnt/β-catenin signalling pathway activator or with an inhibitor of a Wnt/β-catenin signaling pathway repressor, at different concentrations and during different periods of time before transplantation of the so treated cells into an animal and then analyzing if cell fusion-mediated reprogramming occurs, for example, by detecting and/or determining the expression of undifferentiated cell markers, e.g., Nanog, Oct4, Nestin, Otx2, Noggin, SSEA-1, etc. By mode of illustration, in a particular embodiment, a range of about 100 to about 300ng/ml of Wnt3a may be used to treat said cells under suitable specific culture conditions. In a particular embodiment, the cells are treated with Wnt3a as Wnt/β-catenin pathway activator, in a suitable amount of about 100-300 ng/µl for 24 h before transplantation of the treated cells.

In another particular embodiment, the cells are treated before transplantation with BIO as an inhibitor of a Wnt/β-catenin signalling pathway repressor (GSK-3), preferably in a range of about 1 µM to 10 mM of BIO, preferably are treated for 24 hours.

In another particular embodiment, the cell or cell population for use according to the invention overexpresses a Wnt/β-catenin pathway activator and/or an inhibitor of a Wnt/β-catenin pathway repressor.

As used herein, a "cell that overexpresses a Wnt/β-catenin signalling pathway activator" is a cell, such as a cell selected from the group consisting of a HSC, a hematopoietic progenitor cell and a MSC, that has been genetically manipulated to overexpress a Wnt/β-catenin signalling pathway activator, wherein said Wnt/β-catenin signalling pathway activator is a peptide or protein. In a particular embodiment, said Wnt/β-catenin signalling pathway activator is a Wnt protein isoform such as Wnt1, Wnt2, Wnt2b/13, Wnt3, Wnt3a, Wnt4, Wnt5a, Wnt5b, Wnt6, Wnt7a, Wnt8a, Wnt8b, Wnt9a, Wnt9b, Wnt10a, Wnt10b, Wnt11, Wnt16, or a functional variant or fragment thereof. In another particular embodiment, said Wnt/β-catenin signalling pathway activator is β-catenin or a functional variant or fragment thereof. In another particular embodiment, said Wnt/β-catenin signalling pathway activator is a R-spondin such as R-spondin-1, R-spondin-2, R-spondin-3, R-spondin-4, or a functional isoform, variant or fragment thereof.

As used herein, a "cell that overexpresses an inhibitor of the Wnt/β-catenin signalling pathway repressor" is a cell, such as a cell selected from the group consisting of a HSC, a hematopoietic progenitor cell and a MSC, that has been genetically manipulated to overexpress an inhibitor of a Wnt/β-catenin signalling pathway repressor, wherein said Wnt/β-catenin signalling pathway activator is a peptide or protein. Illustrative non-limitative examples of repressors are Axin, CK1, APC, DKK and GSK3.

In an embodiment, the polynucleotide comprising the nucleotide sequence encoding the Wnt/β-catenin signalling pathway activator or the inhibitor of a Wnt/β-catenin signalling pathway repressor is comprised in an expression cassette, and said polynucleotide is operatively bound to (i.e., under the control of) an expression control sequence of said polynucleotide comprising the nucleotide sequence encoding the Wnt/β-catenin signalling pathway activator or the inhibitor of a Wnt/β-catenin signalling pathway repressor. Expression control sequences are sequences that control and regulate transcription and, where appropriate, translation of a protein, and include promoter sequences, sequences encoding transcriptional regulators, ribosome binding sequences (RBS) and/or transcription terminator sequences. In a particular embodiment, said expression control sequence is functional in eukaryotic cells, such as mammalian cells, preferably human cells, for example, the human cytomegalovirus (hCMV) promoter, the combination of the cytomegalovirus (CMV) early enhancer element and chicken beta-actin promoter (CAG), the eukaryotic translation initiation factor (elF) promoter, etc.

Advantageously, said expression cassette further comprises a marker or gene encoding a motive or for a phenotype allowing the selection of the host cell transformed with said expression cassette. Illustrative examples of said markers that could be present in the expression cassette of the invention include antibiotic-resistant genes, toxic compound-resistant genes, fluorescent marker-expressing genes, and generally all those genes that allow selecting the genetically transformed cells. The gene construct can be inserted in a suitable vector. The choice of the vector will depend on the host cell where it will subsequently be introduced. By way of illustration, the vector in which the polynucleotide comprising the nucleotide sequence encoding the Wnt/β-catenin signalling pathway activator is introduced can be a plasmid or a vector which, when introduced in a host cell, either becomes integrated or not in the genome of said cell. Said vector can be obtained by conventional methods known by persons skilled in the art. In a particular embodiment, said recombinant vector is a vector that is useful for transforming animal cells, preferably mammalian cells. Said vector can be used to transform, transfect or infect cells such as cells selected from the group consisting of HSCs, hematopoietic progenitor cells and MSCs. Transformed, transfected or infected cells can be obtained by conventional methods known by persons skilled in the art.

The cell or cell population for use in the treatment of Parkinsonism according to the invention, preferably isolated cells, may be used to initiate, or seed, cell cultures. The specific cells may be isolated in view of their markers as it has been previously mentioned. Isolated cells may be transferred to sterile tissue culture vessels, either uncoated or coated with extracellular matrix or ligands such as laminin, collagen (native, denatured or crosslinked), gelatin, fibronectin, and other extracellular matrix proteins. The cells for use in the treatment of a Parkinsonism according to the invention may be cultured in any suitable culture medium (depending on the nature of the cells) capable of sustaining growth of said cells such StemSpan™ Serum-Free Expansion Medium (SFEM) that has been developed for the *in vitro* culture and expansion of human hematopoietic cells, in addition stem cell factor (SCF) and erythropoietin; cytokines as interleukin-3 (IL-3), interleukin-6 (IL-6), FMS-like tyrosine kinase 3 (Flt3). The HSPCs may be cultured in the presence of an AF2024 mouse liver fibroblast feeder layer, which is known to support the growth of human hematopoietic stem cells. The cells may be seeded in culture vessels at a density to allow cell growth.

Methods for the selection of the most appropriate culture medium, medium preparation, and cell culture techniques are well known in the art.

In a another preferred embodiment, the cell or the cell population for use according to the invention, after transplantation into the brain of the subject, are fused with a neuron or with a glial cell from said subject, preferably with a glial cell, whereby a hybrid cell is formed and reprogramming of said hybrid cell is induced.

"Neuron", as used herein, relates to an electrically excitable cell that processes and transmits information through electrical and chemical signals. In a preferred embodiment the neuron is a dopaminergic neuron.

"Glial cell", as used herein, relates to a non-neuronal cell that maintains homeostasis, forms myelin, and provides support and protection for neurons in the central nervous system and peripheral nervous system.

The term "cell fusion", as used herein, relates to cell-cell fusion that occurs spontaneously or mediated by exogenous agents

"Hybrid cell", as used herein, relates to a cell produced by the fusion of two different cells, preferably a stem cell and a somatic cell. In the context of the present invention, the hybrid cell is a cell produced by a fusion between a cell or cell population of the invention and neurons or glial cells. The hybrid cell formed by a fusion with a neuronal cell may be identified by detecting the dopaminergic neuron marker tryrosine hydrolase (TH+) or the neuronal nucleus protein (NeuN) by any method known in the art.

"Tyrosine hydrolase" (TH), as used herein, relates to an enzyme responsible for catalyzing the conversion of the amino acid L-tyrosine to L-3,4-dihydroxyphenylalanine (L-DOPA). The complete sequence of human TH has the UniProt accession number P07101 (March 16, 2016).

"Neuronal nucleus protein" (NeuN) or FoX-3, as used herein, relates to a neuronal nuclear antigen that is commonly used as a biomarker for neurons. The complete sequence of human NeuN has the UniProt accession number A6NFN3 (March 16, 2016).

Alternatively, the hybrid cell formed by fusion with a glial cell may be identified by detecting the expression of GFAP, or the macrophage/ microglia marker CD11b.

"GFAP" refers to glial fribrillary acidic protein, an intermediate filament (IF) protein. The complete sequence of human GFAP has the UniProt accession number P14136 (March 16, 2016).

"CD11 b" has been previously defined.

"Reprogramming", as used herein, refers to the de-differentiation of a somatic cell (i.e. the passage of a neuron or a glial cell from the differentiated state, wherein it cannot generate other types of cells, to an undifferentiated state wherein it retains the potential to give rise to specialized cells) which is followed by differentiation of the hybrid cell previously formed as a result of the cell fusion between a stem cell (e.g., a HSC, a hematopoietic progenitor cell or a MSC) and a somatic cell (e.g., a neuron or a glial cell).

Reprogramming may be detected by determining a change in morphology (Acquistapace et al., 2011) or detecting changes in some markers as described in the experimental section.

### Pharmaceutical composition

In another aspect, the invention relates to a pharmaceutical composition selected from the group consisting of:
1) a pharmaceutical composition comprising a cell selected from the group consisting of a hematopoietic stem cell (HSC), a hematopoietic progenitor cell (HPC), and a mesenchymal stem cell (MSC), or a cell population comprising, consisting essentially of or consisting of any combination thereof, and a pharmaceutically acceptable carrier;
2) a pharmaceutical composition comprising a cell selected from the group consisting of a hematopoietic stem cell (HSC), a hematopoietic progenitor cell (HPC), and a mesenchymal stem cell (MSC), or a cell population comprising, consisting essentially of or consisting of any combination thereof, wherein the Wnt/β-catenin signaling pathway of said cells is activated, and a pharmaceutically acceptable carrier; and
3) a pharmaceutical composition comprising a cell selected from the group consisting of a hematopoietic stem cell (HSC), a hematopoietic progenitor cell (HPC), and a mesenchymal stem cell (MSC), or a cell population comprising, consisting essentially of or consisting of any combination thereof, a Wnt/β-catenin signalling pathway activator or an inhibitor of a Wnt/β-catenin signalling pathway repressor, and a pharmaceutically acceptable carrier,
for use in the treatment of Parkinsonism.

In another preferred embodiment, the pharmaceutical composition for use according to the invention further comprises a neurotrophic factor, more preferably BDNF and/or GDNF.

As used herein, the term "carrier" includes vehicles, media or excipients, whereby the cell or cell population can be administered. Obviously, said carrier must be compatible with said cells. Illustrative, non-limiting examples of suitable pharmaceutically acceptable carriers include any physiologically compatible carrier, for example, isotonic solutions (e.g., 0.9% NaCl sterile saline solution, phosphate buffered saline (PBS) solution, Ringer-lactate solution, etc.) optionally supplemented with serum, preferably with autologous serum; cell culture media (e.g., DMEM, etc.); etc. or, alternatively, a solid, semisolid, gelatinuos or viscous support medium such as collagen, collagen-glycosaminoglycan, fibrin, polyvinyl chloride, poly(amino acids) such as polylysine, polyornithine, etc., hydrogels, agarose, silicone dextran sulfate. Likewise, if desired, the support medium can, in specific embodiments, contain growth factors or other agents. If the support is a solid, semisolid, or gelatinous support, the cells of the invention can be introduced into a liquid phase of the carrier which is subsequently treated such that it becomes a more solid phase.

The pharmaceutical composition of the invention may comprise auxiliary components as would be familiar to medicinal chemists or biologists, for example, a viscosity enhancing agent (e.g. hydroxyethylcellulose, hydroxypropylcellulose, methylcellulose, polyvinylpyrrolidone, etc.), etc. In some embodiments, the pharmaceutical composition of the invention may contain a preservative (e.g., benzalkonium chloride, benzethonium chloride, chlorobutanol, phenylmercuric acetate or nitrate, thimerosal, methyl or propylparabens, etc.). Said pharmaceutically acceptable substances which can be used in the pharmaceutical composition of the invention are generally known by the persons skilled in the art and are normally used in the preparation of cell compositions. Examples of suitable pharmaceutical carriers are described, for example, in "Remington's Pharmaceutical Sciences", of E.W. Martin.

The pharmaceutical composition of the invention, if desired, can also contain, when necessary, additives to increase, control or give rise, in another manner, to the desired therapeutic effect of the cell or cell population of the invention which comprise said pharmaceutical composition, and/or auxiliary substances or pharmaceutically acceptable substances such as buffering agents, surfactants, cosolvents, preservatives, etc.

The pharmaceutical composition of the invention will contain a prophylactic or pharmaceutically effective amount of the cells of the invention, or of the cell population of the invention to provide the desired therapeutic effect. In the sense used in this description, the term "therapeutically or prophylactically effective amount" relates to the amount of cells of the invention, or of the cell population of the invention contained in the pharmaceutical composition of the invention which is capable of producing desired therapeutic effect and will generally be determined by, among other factors, the characteristics of said cells themselves and the desired therapeutic effect. Generally, the therapeutically effective amount of said cell or cell population of the invention that must be administered will depend on, among other factors, the characteristics of the subject himself, the seriousness of the disease, the dosage form, etc. For this purpose, the dose mentioned in this invention must only be taken into account as a guideline for the person skilled in the art, who must adjust this dose depending on the aforementioned factors. In a particular embodiment, the pharmaceutical composition of the invention is administered in a dose containing between approximately 1x10⁵ and approximately 1x10⁸ cells or cell population of the invention per kilogram (kg) of body weight of the receiver, typically, about 10⁶-10⁷ cells or cell population of the invention per inoculation, on a weekly, fortnightly or monthly basis, and with an inoculation range comprised between 3 and 6 inoculations per patient. In a specific embodiment, by way of non-limiting illustrative example, the pharmaceutical composition of the invention can be administered as a single dose containing between approximately 1x10⁵ and approximately 10x10⁸ cells or cell population of the invention per kg of body weight of the receiver, preferably between approximately 5x10⁵ and approximately 5x10⁷ cells or cell population of the invention per kg of body weight of the receiver, more preferably between approximately 1x10⁶ and approximately 1x10⁷ cells or cell population of the invention per kg of the body weight of the receiver depending on the aforementioned factors. In another preferred embodiment, the dose is 3-8x10⁶ cells. The dose of cells or cell population of the invention can be repeated depending on the status and evolution of the subject in temporal intervals of days, weeks or months that must be established by the specialist in each case.

The pharmaceutical composition of the invention will be formulated according to the selected method of administration. The pharmaceutical composition of the invention can be prepared in a gel or liquid dosage form, for example, in the form of suspension to be injected or perfused into a subject. Non-limiting illustrative examples include formulating the pharmaceutical composition of the invention in a sterile suspension with a pharmaceutically acceptable excipient such as an isotonic solution, for example, phosphate buffered saline (PBS) solution, or any other suitable pharmaceutically acceptable carrier for administration into a subject.

In a preferred embodiment, the pharmaceutical composition is transplanted or administered into the brain, preferably into the midbrain, more preferably, above the *substantia nigra* pars compacta (SNpc), of a subject in need of treatment.

In another preferred embodiment, the cell population contained in the pharmaceutical composition comprises a hematopoietic stem cell (HSC) and a hematopoietic progenitor cell (HPC).

The pharmaceutical composition of the invention, if desired, can be stored until its time of use by means of conventional processes known by the person skilled in the art. This pharmaceutical composition can also be stored together with additional medicaments useful in the treatment of diseases, in an active form comprising a combined therapy. For short term storage (less than 6 hours), the pharmaceutical composition of the invention can be stored at or below room temperature in a sealed container, complementing it or not with a nutrient solution. Medium term storage (less than 24 hours) is performed preferably at 2-8°C and the pharmaceutical composition of the invention will include a buffered iso-osmotic solution in a container made up of or covered with a material which prevents cell adhesion. Long term storage is preferably carried out by means of suitable cryopreservation and storage in conditions which promote the retention of cell function

All terms and embodiments previously described in the first aspect of the invention are equally applicable to the pharmaceutical composition for use in the treatment of Parkinsonism according to the invention.

### Kit

In another aspect, the invention relates to a kit selected from the group consisting of:
1) a kit comprising a cell population comprising a cell selected from the group consisting of a hematopoietic stem cell (HSC), a hematopoietic progenitor cell (HPC), and a mesenchymal stem cell (MSC), or a cell population comprising any combination thereof, and instructions for use of the kit components;
2) a kit comprising a cell population comprising a cell selected from the group consisting of a hematopoietic stem cell (HSC), a hematopoietic progenitor cell (HPC), and a mesenchymal stem cell (MSC), or a cell population comprising any combination thereof, wherein the Wnt/β-catenin signaling pathway of said cells is activated, and instructions for use of the kit components; and
3) a kit comprising a cell population comprising a cell selected from the group consisting of a hematopoietic stem cell (HSC), a hematopoietic progenitor cell (HPC), and a mesenchymal stem cell (MSC), or a cell population comprising any combination thereof, a Wnt/β-catenin signalling pathway activator or an inhibitor of a Wnt/β-catenin signalling pathway repressor, and instructions for use of the kit components,
for use in the treatment of Parkinsonism.

Alternatively, the invention relates to the use of a kit selected from the group consisting of:
1) a kit comprising a cell population comprising a cell selected from the group consisting of a hematopoietic stem cell (HSC), a hematopoietic progenitor cell (HPC), and a mesenchymal stem cell (MSC), or a cell population comprising any combination thereof, and instructions for use of the kit components;
2) a kit comprising a cell population comprising a cell selected from the group consisting of a hematopoietic stem cell (HSC), a hematopoietic progenitor cell (HPC), and a mesenchymal stem cell (MSC), or a cell population comprising any combination thereof, wherein the Wnt/β-catenin signaling pathway of said cells is activated, and instructions for use of the kit components; and
3) a kit comprising a cell population comprising a cell selected from the group consisting of a hematopoietic stem cell (HSC), a hematopoietic progenitor cell (HPC), and a mesenchymal stem cell (MSC), or a cell population comprising any combination thereof, a Wnt/β-catenin signalling pathway activator or an inhibitor of a Wnt/β-catenin signalling pathway repressor, and instructions for use of the kit components,
for the preparation of a medicament for the treatment of Parkinsonism.

Alternatively, the invention relates to a method for the treatment of Parkinsonism in a subject comprising the use of a kit selected from the group consisting of:
1) a kit comprising a cell population comprising a cell selected from the group consisting of a hematopoietic stem cell (HSC), a hematopoietic progenitor cell (HPC), and a mesenchymal stem cell (MSC), or a cell population comprising any combination thereof, and instructions for use of the kit components;
2) a kit comprising a cell population comprising a cell selected from the group consisting of a hematopoietic stem cell (HSC), a hematopoietic progenitor cell (HPC), and a mesenchymal stem cell (MSC), or a cell population comprising any combination thereof, wherein the Wnt/β-catenin signaling pathway of said cells is activated, and instructions for use of the kit components; and
3) a kit comprising a cell population comprising a cell selected from the group consisting of a hematopoietic stem cell (HSC), a hematopoietic progenitor cell (HPC), and a mesenchymal stem cell (MSC), or a cell population comprising any combination thereof, a Wnt/β-catenin signalling pathway activator or an inhibitor of a Wnt/β-catenin signalling pathway repressor, and instructions for use of the kit components,

Therefore, in the method of the invention, the disclosed kits are used for the treatment of Parkinsonism.

In a preferred embodiment, the kit for use in the present invention comprises a neurotrophic factor, more preferably BDNF and/or GDNF.

In the context of the present invention, "kit" is understood as a product containing the different products necessary for treating Parkinsonism packed so as to allow their transport and storage. Materials suitable for packing the components of the kit include crystal, plastic (polyethylene, polypropylene, polycarbonate and the like), bottles, vials, paper, envelopes and the like. Additionally, the kits of the invention can contain instructions for the simultaneous, sequential or separate use of the different components which are in the kit. Said instructions can be in the form of printed material or in the form of an electronic support capable of storing instructions such that they can be read by a subject, such as electronic storage media (magnetic disks, tapes and the like), optical media (CD-ROM, DVD) and the like. Additionally, or alternatively, the media can contain Internet addresses that provide said instructions.

In a particular aspect, the kit of the invention further comprises a device for administering cells. Said devices include, but are not limited to, syringes, injection devices, catheters, trocars, cannulae and stents.

All terms and embodiments previously described are equally applicable to the kit for use in the treatment of Parkinsonism according to the invention.

The invention is detailed below by means of the following examples which are merely illustrative and by no means limiting for the scope of the invention.

### EXAMPLES

### Materials and methods

**Mice**. The mice were housed in accordance with the Ethical Committee for Animal Experimentation of the Government of Catalonia, and the experiments were performed in accordance with the rules set by the local Animal Ethics Committee. All of the mice in this study were generated in a wild-type C57BL/6J background. The inventors used wild-type C57BL/6J and the following transgenic mice: CAG-Cre (Hayashi and McMahon, 2002), β-actin-Cre (Srinivas et al., 2001), CAG-RFP (Long et al., 2005), GFAP-Cre (Gregorian et al., 2009), FoxA2-Cre (Park et al., 2008), R26Y (Srinivas et al., 2001), and ROSA26iDTR (Buch et al., 2005).

**Establishment of the MPTP / 60HDA mouse models.** For the 1-methyl-4-phenyl-1, 2,3,6-tetrahydropyridine (MPTP) mouse model, eight- to 12-week-old male mice received one intraperitoneal injection of MPTP-HCl per day (30 mg/kg free base; Sigma) for five consecutive days, according to the sub-acute MPTP injection paradigm (Vila et al., 2001). Control mice received 0.9% sterile saline injections only. For the 6-hydroxydopamine (6OHDA) mouse model, either male or female mice had 6OHDA injected into the right substantia nigra (SNpc) pars compacta (Parish et al., 2001), under anesthesia and analgesia (2% isoflurane in 2:1 oxygen/ nitrous oxide), using a Kopf stereotaxic frame (Kopf Instruments) and a 5 µl Hamilton syringe fitted with a fine capillary. The 6OHDA was used at 1.5 µg/µl (calculated as the free base; Sigma) dissolved in a solution of 0.2 mg/ml ascorbic acid in 0.9% sterile saline. A single injection of 2 µl was performed using the stereotaxic coordinates according to Paxinos and Franklin (2008): -3.0 mm anterior/ posterior, 1.05 mm lateral with respect to the bregma, and -4.7 mm ventral from the dura, with a flat skull position. Injections were made at a rate of 0.25 µl/min with a further 4 min allowed for the toxin to diffuse before slow withdrawal of the capillary, followed by cleaning and suturing of the wound.

**Cell preparation and transplantation.** Just before the transplantation, lineage-negative HSPCs were isolated from the total bone marrow of donor mice using Lineage Cell Depletion kits (Miltenyi Biotech). At day 3 after the last MPTP injection or the 6OHDA infusion, the recipient mice were placed into a Kopf stereotaxic frame and received one injection of approximately 60000 cells suspended in 2 µl phosphate-buffered saline (PBS) above the right substantia nigra (stereotaxic coordinates were determined according to Paxinos and Franklin (2008): -2.9 mm anterior, 1.3 mm lateral with respect to the bregma, and -4.5 mm ventral from the dura, with a flat skull position. The intracerebral injection was performed using a 5 µl Hamilton microsyringe coupled with a 33-gauge needle. Cell infusions were performed at a rate of 0.5 µl/min. On completion of the injection, the needle was left in place for 4 min before being retracted slowly at a rate of 1 mm/min, to avoid reflux along the injection track. After the surgery, the mice were placed under a warm lamp until their complete awakening. To block the Wnt/β-catenin signaling by infusion of Dckkopf-1 (Dkk1) into the SNpc in vivo, the recombinant Dkk1 protein (R&D Systems, MN, USA) was dissolved in sterile physiological saline (0.9% NaCl) at a final concentration of 1 µg/µl. One infusion of Dkk1 was carried out unilaterally into the SNpc using a 5 µl Hamilton microsyringe and 2 µg/ infusion at the same time of the cell transplantation.

**Perfusion and immunofluorescent analysis by microscopy**. The mice were euthanized with CO2 in an appropriate chamber to examine the phenotypes of the transplanted cells. The mice were perfused with 4% paraformaldehyde in 0.1 M PBS. The brains were removed and post-fixed in 4% paraformaldehyde overnight at 4 °C. The following day, the brains were equilibrated in 30% sucrose and PBS solution for 48 h, frozen in 2-methylbutane, and stored at -80 °C. Coronal sections were cut serially through the whole brain at a 30 µm thickness and stored free-floating in cryo-storage solution (30% ethylene glycol, 30% glycerol, 0.5 M phosphate buffer, pH 7.4). For immunofluorescence staining, the sections were rehydrated and blocked using goat serum. The sections were incubated with the primary antibodies to GFP (1:1000), RFP (1:1000), GFAP (1:1000), Cre (1:1000), CD11 b (1:300), TH (1:1000), NeuN (1:1000), GAD65/67 (1:500), GABA (1:250) from Millipore, or to CD68 (1:100, AbD Serotec), Iba-1 (1:1000, Wako), Olig2 (1:300, antibody project group, CRG) Sox2 (1:500, Abcam), GFP (1:500, Invitrogen), S100β (1:500, Sigma), or CNPase (1:100) or FoxA2 (1:100) from Santa Cruz, The sections were then washed and labeled with the fluorescent secondary antibody (1:1000, Invitrogen). The antibody against Wnt1 (1:100, Abcam) required antigen retrieval as follows: free floating sections were incubated for 30 min in a 10 mM sodium citrate solution (pH 8.5-9.0) preheated to and maintained at 80 °C in a water-bath, followed by a 30-min incubation in blocking serum, to reduce nonspecific staining. Fluorescence microscopy analysis and image acquisition were carried out with a confocal laser-scanning microscope (Leica TCS-SP5). For the immunohistochemical analysis, coronal sections were cut serially 30 µm through the midbrain and through the striatum (CPu: Caudate-putamen). From each mouse, four series of sections containing every fourth section were prepared for the midbrain (included the substantia nigra), whereas six series of every sixth section were prepared for the striatum. Anatomical landmarks were determined according to Paxinos and Franklin (2008). The standard avidin-biotin complex method was used to immunostain the brain sections. Briefly, the sections were treated for 30 min with goat serum in PBS containing 0.3% Triton X-100, and then incubated with a rabbit anti-TH antibody (1:2000, Millipore) for 48 h at 4 °C. The sections were rinsed in PBS and incubated with a biotinylated goat anti-rabbit secondary antibody (1:500) for 2 h, followed by avidin-biotin peroxidase complex (ABC Elite kit; Vector Laboratories) at room temperature for 2 h. The chromogen used was 3,3'-diaminobenzidine tetrahydrochloride (DAB Substrate kits; Vector Laboratories). The DAB-stained striatum sections were used for optical density measurement, whereas midbrain sections were counterstained with cresyl violet (Nissl) and used for stereology.

**Unbiased Stereological cell counting and optical density measurements**. Blinded observers performed all of the assessments. Immunohistochemical staining of the midbrain was examined (n=12 sections/ animal) using an optical microscope (Leica, France) equipped with the CAST semiautomatic stereological software (VisioPharm). Then, the total numbers of neurons stained for TH and Nissl in the substantia nigra were counted stereologically. TH neuron fibers in the striatum were analyzed according to optical density measurements (n=6 sections/ animal). The Image J processing software (National Institutes of Health) was used to analyze the images. These were thresholded to limit measurements to the TH+ fibers, and then an integrated density measurement was obtained by multiplying the TH-stained area by the mean pixel intensity. The TH neuron counts and the optical density of their fibers are expressed as means ±s.e.m. of the percentages of the TH+ cell counts and fibers of the saline controls.

Enzymatic midbrain cell dissociation and FACS sorting of transplanted **cells.** The transplanted side of the midbrain was dissected out and dissociated for 30 min at 37 °C with the enzyme papain (Papain Dissociation System, Worthington Biochem) in Earl's balanced salt solution with DNase, according to the manufacture protocol. Brain slices were triturated with three glass pipettes of decreasing tip diameters, and the dissociated cells were centrifuged at 300xg for 5 min at room temperature. To remove excess debris, the cell pellets were resuspended in Earl's balanced salt solution, DNase, and albumin ovomucoid inhibitor, and the cell suspension was subjected to centrifugation on an albumin ovomucoid inhibitor discontinuous gradient (according to the Papain Dissociation System protocol) at 300xg for 6 min at 4 °C. The cell pellets were resuspended in the FACS buffered media and filtered through 70 µm mesh. The cells were then treated with DAPI (Sigma, St. Louis, MO, USA) to label the dead cells, and analyzed on a BD LSRII or sorted on a BD FACSAria II sorting machine (Becton Dickinson) for FITC signals (to detect the YFP) and PE signals (to detect the RFP). The cells were kept on ice pre-sorting and post-sorting. A polyclonal antibody against Diphtheria toxin-FITC (1:50, Acris) was used to detect the Diphtheria toxin receptor and for the FACS analysis of the RFP+DTR+ cells.

**Gene expression analysis.** The total RNA was extracted using RNA Isolation Micro kits (Qiagen), according to the manufacturer protocol. The eluted RNA was reverse-transcribed with SuperScript III (Invitrogen) and real-time qPCR reactions using SYBER Green I Master (Roche) were performed in a LightCycler® 480 System (Roche), according to the manufacturer recommendations. The species-specific oligos used are listed in Table II.

**Table II. Primers**

| **Target protein** | **Direction** | **Mouse-specific primers for qRT-PCR** | **SEQ ID NO:** |
|---|---|---|---|
| β-Actin | Forward | ACGTTGACATCCGTAAAGACCT | 1 |
| | Reverse | GCAGTAATCTCCTTCTGCATCC | 2 |
| CD34 | Forward | TGGTACTTCCAGGGATGCT | 3 |
| | Reverse | TGGGTAGCTCTCTGCCTGAT | 4 |
| Oct4 | Forward | CGTGGAGACTTTGCAGCCTG | 5 |
| | Reverse | GCTTGGCAAACTGTTCTAGCTCCT | 6 |
| GFAP | Forward | GGAGACGCATCACCTCTGCGC | 7 |
| | Reverse | AGAAATCCACCCGGGTCGGG | 8 |
| Nestin | Forward | TGGAAGTGGCTACA | 9 |
| | Reverse | TCAGCTTGGGGTCAGG | 10 |
| Axin2 | Forward | GGGAGCAGTTTTGTGGCAGCA | 11 |
| | Reverse | AGGGTCCTGGGTAAATGGGTGAG | 12 |
| Wnt5a | Forward | TGCGGAGACAAACATCGACTA | 13 |
| | Reverse | AAGTTCATGAGGATGCGTGC | 14 |
| Fzd1 | Forward | GTCGGTTTCAAGGCTCCTCC | 15 |
| | Reverse | CCTCCAACGGGTTTCTAGGC | 16 |
| Runx1 | Forward | CACCTGTCTCTGCATCGCAGGACT | 17 |
| | Reverse | CCATCCGTGACA-GATACGCACCTC | 18 |
| Sox2 | Forward | CAGGAGAACCCCAAGATGCACAA | 19 |
| | Reverse | AATCCGGGTGCTCCTTCATGTG | 20 |
| Olig2 | Forward | CAAATCTAATTCACATTCGGAAGGTTG | 21 |
| | Reverse | GACGATGGGCGACTAGACACC | 22 |
| MAP2 | Forward | TTGGTGCCGAGTGAGAAGA | 23 |
| | Reverse | GTCTGGCAGTGGTTGGTTAA | 24 |
| FoxA2 | Forward | CCCTACGCCAACATGAACTCG | 25 |
| | Reverse | GTTCTGCCGGTAGAAAGGGA | 26 |
| NeuN | Forward | GGCAATGGTGGGACTCAAAA | 27 |
| | Reverse | GGGACCCGCTCCTTCAAC | 28 |
| Wnt1 | Forward | GGTTTCTACTACGTTGCTACTGG | 29 |
| | Reverse | GGAATCCGTCAACAGGTTCGT | 30 |
| Caspase-9 | Forward | TGGGTCTCGGCGGGATCAGG | 31 |
| | Reverse | GCGTGTGCTGAGCCCTGAGG | 32 |
| GATA-4 | Forward | GGCCTCTACCACAAGATGAA | 33 |
| | Reverse | ACTGCCAGACTACCACCACC | 34 |
| Brachyury | Forward | GCTTCAAGGAGCTAACTAACGAG | 35 |
| | Reverse | CCAGCAAGAAAGAGTACATGGC | 36 |
| Sox7 | Forward | CAGCAAGATGCTGGGAAAG | 37 |
| | Reverse | TGCATCATCCACATAGGGTCT | 38 |
| BMP4 | Forward | GAGGGATCTTTACCGGCTCC | 39 |
| | Reverse | GTTGAAGAGGAAACGAAAAGCAG | 40 |

The qPCR data were normalized to the expression of β-Actin. The data are means of three independent experiments, each carried out in triplicate.

**RNA extraction and real-time PCR.** The mice were sacrificed by cervical dislocation at the indicated times after PBS or HSPC transplantation. Their brains were rapidly removed, and the ventral midbrain (which included the SNpc) was dissected out on chilled plates and immediately flash frozen in liquid nitrogen. Then, the samples were cut and divided in two equal parts for subsequent RNA and protein processing. RNA extraction was carried out in tissue samples homogenized using the RNeasy Mini kits (Qiagen). One microgram of RNA was used for reverse transcription with Superscript III (Invitrogen). LightCycler 480 SYBER Green I Master (Roche) was used with a LightCycler® 480 System (Roche) for quantitative RT-PCR. The samples were assayed in triplicate, and the data were normalized to the relative amounts of β-actin, with interpretation using the ΔCT method. The final data are expressed as fold-changes following normalizing of the data to the control values.

**Microarray analysis.** RFP+ and RFP+YFP+ cells were FACS sorted from transplanted mouse brain samples. Samples from 2 mice were pooled for each replicate, for 2 replicates. The total RNA was extracted with RNAClean XP kits(Agentcourt A63987), following the manufacturer instructions. It was then immediately amplified using TransPlex Complete Whole Transcriptome Amplification kits (Sigma WTA2), to generate the cDNA. Then, this was labeled and hybridized to the Agilent SurePrint G2 Mouse Gene Expression 8x60K microarray (G4852A, ID 028005).

**Preprocessing of data.** Scanning was carried out and the intensity data of each individual hybridization was extracted and the quality was assessed with the Feature Extraction software 10.7 (Agilent). The raw data was taken from the Feature Extraction output files and was corrected for background noise using the normexp method (Ritchie et al., 2007). To assure comparability across samples, we used quantile normalization. Differential expression analysis was carried out on noncontrol probes and log2-transformed data with an empirical Bayes approach on linear models (LIMMA). The data were corrected for multiple testing according to the false discovery rate (FDR) method. All of the statistical analyses were performed with the Bioconductor project in the R statistical environment.

**Enrichment analysis.** The list of enriched genes represented by the probes that exceeded a 1.1-fold change was used as the input into Enrichr tools. The enrichment terms were scored by p-value, Z-score, and combined score, and are shown in the Figures as sorted by p-value. Adjustment for multiple comparisons was applied (p adj. function in R) and the data were filtered using two cut-offs: p <0.05 and Z-score <-1.5. The Z-score applies a correction to the Fisher exact test p value that Enrichr developed, to correct for possible false-positive results from large gene sets that give lower p values with large gene overlap.

**Gene set enrichment analysis.** Pre-processing of the gene list for use in the gene set enrichment analysis (GSEA) was performed as follows: for duplicated genes, the copy with the highest absolute log2 ratio was kept, and the remaining ones were discarded. Mouse gene symbols were then converted to human gene symbols using the Jackson laboratory homology data, as indicated in the GSEA documentation. GSEA was used in GSEAPreranked mode on "PARK_HSC_AND_MULTIPOTENT_PROGENITORS" and "NEUROGENESIS" GSEA gene sets from the Molecular Signature Database (MsigDB, systematic names M1456 and M11351, respectively). The magnitude of normalized enrichment scores (NES) and false discovery rate (FDR) values were used to evaluate each analysis. The FDR is the estimated probability that a gene set with a given NES represents a false positive finding. FDR of ≤25% is a standard cut-off for GSEA analysis indicating that the probability of a false enrichment is below 25% (Subramanian et al., 2005).

**Western blotting.** The protein extracts were prepared by homogenizing the tissue in RIPA buffer (Sigma) supplemented with protease inhibitors (Roche). Then, the protein concentrations were determined using the Bradford assay (Bio-Rad). The proteins were denatured, reduced, separated by SDS/PAGE, and transferred to nitrocellulose membranes (Protran; Whatman, Schleicher & Schuell). The membranes were blocked with 5% nonfat dry milk in TBS-Tween (TBST) for 60 min, incubated with the primary antibodies overnight at 4 °C, washed three times with TBST for 10 min, incubated with the peroxidase-conjugated secondary antibody in TBST with 2.5% nonfat dry milk for 60 min, and washed three times with TBST for 10 min. Immunoreactive proteins were detected using Super signal West Dura HRP Detection kits (Pierce). The antibodies used were against active β-Catenin (1:1000, Millipore), GFAP (1:2000, Millipore), and Wnt1 (1:1000, Abcam). β-Actin was used as an internal control for the equal input of the protein samples, using an anti-β-actin antibody (1:1000, Abcam). The Western blotting bands were quantified using the ImageJ software, by measuring the band intensity (Area x OD) for each group, and normalizing to β-actin. The final data are expressed as fold-changes with the data normalized to the control values.

**Tamoxifen treatment for Cre-recombinase induction and diphtheria toxin injection.** To induce Cre recombination for the YFP labeling, 20 mg tamoxifen (T5648, Sigma) was dissolved in 100 µl ethanol and 900 µl corn oil at 56 °C for 2-3 h. Tamoxifen was stored in the dark at 4 °C until use, for up to 3 weeks. Tamoxifen was warmed to 37 °C before administration. The mice received intraperitoneal injections of a total 75 mg tamoxifen/kg body weight, once per day for three consecutive days. To ablate the hybrid cells, iDTR transgenic mice were injected intraperitoneally with 525 ng diphtheria toxin (D0564, Sigma-Aldrich, St. Louis, MO, USA) in 300 ml PBS per injection, for four consecutive days.

**Neurochemical analysis.** Tissue DA, DOPAC and HVA content were determined on tissue samples from striatum (CPu) and cortex (Ctx) by HPLC with electrochemical detection (Waters model 2465; +0.7V) as previously described (Bortolozzi & Artigas, 2003). Mice were killed and their brains were quickly removed and placed over a cold plate. Caudate putamen (CPu) and cortex were carefully dissected out, weight, frozen on dry ice and kept at -80°C until assayed. The tissues were homogenized in 200 µl of buffer containing 0.4M perchloric acid containing 0.1% sodium metabisulphite, 0.01% EDTA, 0.1% cysteine and centrifuged at 12000g for 30 min. Aliquots of supernatants were then filtered through 0.45 µm filters (Millex, Barcelona, Spain) and analyzed by HPLC as described. The mobile phase consisted of 0.1 M KH2PO4, 1 mM octyl sodium sulphate, 0.1 mM EDTA (pH 2.65) and 18% methanol. DA and their metabolites were separated on a Mediterranea Sea column (C18, 3µm, 10 cm x 6.4 mm) (Teknokroma, ref TR010042, Barcelona, Spain).

**Animal behavior studies.** The motor responses to sensory stimuli in the MPTP-treated mice were measured using the adhesive removal test. Two training trials were performed prior to the surgery, with an adhesive dot sticker (0.6 cm diameter, Avery) placed on the plantar surface of the forelimb. At 4 weeks after Sham or HSPC transplantation of the MPTP-treated mice, the adhesive dot sticker was placed on the forelimb, and the time to make contact and to remove the sticker from the forelimb was recorded. If a mouse did not remove the sticker within 60 s, it received a score of 60 (seconds). The mean data were calculated across three trials, at one per day. Immediately after this test, the spontaneous activity test was run in the cylinder, by placing the mice into a small transparent cylinder (height, 15.5 cm; diameter, 12.7 cm). The spontaneous activity was videotaped for 3 min, and the number of rears and the time spent grooming were measured. To test forepaw akinesia in the 6OHDA mouse model, lesioned mice were randomly assigned to each treatment group, as the Saline group, the control group (6OHDA+Sham), and the group for the transplantation of the HSPCs. To test the spontaneous use of the forelimbs, the cylinder test was applied to the mice from 4 weeks to 8 weeks after the transplantation. The mice were videotaped in the glass cylinder for 3 min, in the light. The cylinder was placed on a piece of glass and a mirror was positioned at an angle beneath the cylinder to allow a clear view of the motor movements along the ground as well as along the walls of the cylinder. The number of contacts during rearing (measured as the number of supporting touches performed against the cylinder wall) was counted for each paw. The data were presented as the percentage of contralateral paw use over the total contacts. The accelerating rotarod test was used to evaluate the locomotor ability of the mice following MPTP, as described previously (Keshet et al., 2007).

**Quantification and Statistical analysis.** A confocal laser-scanning microscope (Leica TCS-SP5) was used to quantify the immunopositive cells in the sections. All of the quantification of the immunostaining was based on analysis of at least five sections per mouse, from at least three mice. Serial coronal brain sections in the region between -2.06 mm and -3.88 mm from the bregma (Paxinos and Franklin, 2008) were selected and used to quantify the stained areas. The incidence of YFP-immunopositive cells was expressed either relative to the DAPI-stained nuclei (series of sections of the midbrain) or to the number of RFP+ cells for the reporter mice. The data are expressed as means ±s.e.m. of the number of tests stated. Statistical comparisons were made using either Student's t-tests for two independent samples or analysis of variance by one-way ANOVA followed by Bonferroni's multiple comparison post-hoc tests, as indicated in the Figure legends. All of the statistical tests and graphical presentations were performed using the Prism 5.0 software (GraphPad, San Diego, CA). P <0.05 was taken as statistically significant.

### Example 1- Transplantation of HSPCs above the SNpc ameliorates dopaminergic neuron loss and function

This example is aimed to determine whether HSPCs can rescue the Parkinson's disease phenotype after their transplantation into the midbrain of the MPTP and 6OHDA mouse models. Thus HSPCs purified from donor mice were unilaterally transplanted above the right SNpc (Figure 1A).

MPTP and 6OHDA treatments can produce a broad range of mild to severe motor alterations in mice. In the MPTP model, the inventors examined sensorimotor deficits using the adhesive removal test and the cylinder test. In MPTP-treated mice transplanted with HSPCs, the removal time was significantly less in comparison to sham mice (Figure 1B). In addition, in the cylinder test, there was significantly higher spontaneous activity in the group of HSPC-transplanted mice, with respect to the saline group (Figure 1B). The rotarod test is generally used when large doses of MPTP are administered, a condition different from the sub-acute regimen of MPTP used in the present study. Nevertheless, the inventors observed motor function recovery in the rotarod test in the group of HSPC-transplanted mice 14 days after transplantation. However, no significant differences were seen between the Sham and HSPC-transplanted mice at 28 days after transplantation, due to the behavioral recovery of the Sham group (Figure 1 G).

Using an unbiased stereology system, the inventors then quantified the number of TH+ immunoreactive cells in the SNpc and the density of TH+ fibers in the striatum, by optical densitometry. Representative immunohistochemical images showed attenuation of dopamine (DA) neuron degeneration after HSPC transplantation (data not shown). Four weeks after transplantation, the percentage of TH+ cells in the sham-transplanted MPTP-lesioned mice remained very low bilaterally, with up to a 42.55±5.36% loss of DA neurons and up to 46.19±4.96% loss of fibers, in comparison with saline-treated animals (Figure 1C). Interestingly, in the group of HSPC-transplanted MPTP-lesioned mice, the loss of TH+ cells in the SNpc was only around 20.19±4.24%, and the density of TH+ fibers in the striatum was around 26.72±1.86%, compared to saline-treated animals. Thus, there was significant reduction in MPTP-induced loss of dopaminergic neurons in HSPC-transplanted mice (Figure 1C). As a control, they transplanted lineage+ (Lin+) cells purified from the bone marrow of donor mice, and the inventors observed that the reduction in dopaminergic neurons was comparable in the Lin+ and Sham-transplanted mice (Figure 1 H).

The inventors then carried out biochemical measures of the levels of DA and its metabolites in the striatum (CPu) and cortex (Ctx) of HSPC-transplanted mice and controls. There was depletion of CPu and Ctx DA and of the 3,4-dihydroxyphenylacetic acid (DOPAC) and homovanillic acid (HVA) DA metabolites after MPTP administration, with the HSPC-transplanted mice showing a trend toward amelioration in terms of DA in both the CPu and Ctx, and DOPAC and HVA in the Ctx (Figure 1I). Interestingly, 4 weeks after transplantation, in the MPTP+HSPC transplanted mice the metabolite to DA ratio (HVA/DA), a parameter associated to DA turnover in dopaminergic terminals, was significantly lower with respect to the MPTP+Sham mice, in both the CPu and Ctx (Figure 1 D). Furthermore, the DOPAC/DA ratio was also significantly diminished in the CPu of HSPC-transplanted mice (Figure 1D). These data suggest that DA synthesis and storage was increased after HSPC transplantation in both the striatum and Ctx.

Studies have reported that 6OHDA-lesioned mice show behavioral impairments more clearly; in addition, the intact side serves as the internal control for the lesioned side and its responsiveness to HSPC transplantation. Therefore, the inventors monitored forepaw akinesia of 6OHDA-lesioned mice from 4 to 8 weeks after HSPC transplantation, using the cylinder test. In line with previous studies, 6OHDA lesion reduced the use of the contralateral paw. Intracerebral HSPC transplantation restored this deficiency in the grafted mice, an effect that remained significant up to at least 4 weeks (Figure 1 E).

The 6OHDA injections resulted in a loss of DA cells down to 41.08±9.00% of control levels and a loss of fibers down to 46.66±12.58% (Figure 1F; compare Saline, Sham, HSPC groups). The Saline group was the contralateral, left hemisphere side, which was not injected with 6OHDA. Interestingly, the 6OHDA effects were ameliorated by intracerebral HSPC transplantation, which successfully prevented 60HDA-induced neurodegeneration of DA cells (DA cells, 23.44±5.05% loss of control levels; fibers, 26.25% ±9.29% loss of control levels; with no statistically significant difference with respect to the Saline group) (Figure 1 F). These data obtained for the 6OHDA mice, as with those obtained in the MPTP model, strongly suggest that intracerebral HSPC transplantation after damage promotes the survival of the endogenous dopaminergic neurons in the SNpc in addition to functional rescue.

### Example 2- HSPCs transplanted above the SNpc can fuse with neurons and glial cells

HSPCs purified from donor mice expressing Cre recombinase under the CAG promoter (HSPCs^{Cre}) were unilaterally transplanted above the right SNpc of Rosa26-LoxP-Stop-LoxP-YFP (R26Y) transgenic mice (Figure 2A). Shortly after transplantation, the fusion events, i.e., the hybrids detected by yellow fluorescent protein expression (YFP+), were found in the SNpc and not in the contralateral non-grafted hemisphere. These YFP+ cells were scattered in the ventral midbrain 24 h after transplantation, and some of them were also positive for the dopaminergic neuron marker tyrosine hydroxylase (i.e., TH+) (data not shown). The MPTP damage greatly increased the fusion of the transplanted cells (Figure 2B).

A marked inflammatory response induced reduction of the hybrids by 7 days after transplantation. An increased density of glial fibrillary acidic protein (GFAP)-positive astroglia delineated the graft, which was positively immunostained for the microglia/ macrophage marker CD11b, and for the activated microglia marker CD68 (data not shown). Corresponding regions in the contralateral hemisphere and in the sham controls showed glial cell accumulation due to the MPTP-induced damage, or to both MPTP and the needle insertion, respectively (data not shown).

The inventors next studied the identity of the fusion partners in the hybrids in the midbrain. Most of the YFP+ cells co-expressed the neuronal nucleus protein (NeuN). A small percentage co-expressed GFAP, or the macrophage/ microglia marker CD11b. None of the YFP+ cells co-expressed the oligodendrocyte marker (CNPase) (Figure 3A. Thus, the majority of the HSPCs can fuse with neurons and some with glial cells. Interestingly, some YFP+ cells in the SNpc co-localized with the ventral midbrain transcription factor (FoxA2) and with TH, both of which are markers of dopaminergic neurons (Figure 3B). TH is down-regulated after MPTP administration, and therefore the inventors might have underestimated the number of YFP+TH+, double-positive, cells. In contrast, the YFP+ cells never co-localized with the GABAergic neuron markers γ-aminobutyric acid (GABA) and glutamic acid decarboxylase (GAD).

### Example 3-Hybrids undergo reprogramming in vivo one week after HSPC transplantation

The inventors next analyzed the presence of the hybrids at different times after the transplantation. HSPCs^{Cre/RFP} (isolated from mice carrying β-Actin-Cre and the CAG-RFP alleles) were unilaterally grafted at day 3 after the last MPTP intraperitoneal injection in R26Y mice (Figure 3F), which corresponds to the peak of apoptotic cell death in this model (Vila et al., 2001). Control mice (injected with saline instead of MPTP) were also grafted. One week after transplantation, there were high numbers of RFP+YFP+ hybrids with round and oval morphologies in the MPTP-treated mice, which were higher than for the saline-treated group (Figure 3C,D). Some hybrids were localized throughout the right ventral midbrain site, close to the SNpc, in the parenchyma, while others showed apparent association with vascular elements.

The change in morphology of the RFP+YFP+ hybrids indicated a possible reprogramming process (Acquistapace et al., 2011). Therefore, the inventors analyzed changes in the transcription profiles of the hybrids (RFP+YFP+) and in the unfused RFP+ cells purified from the ventral midbrain (Figure 3G). In both cell populations, there was almost no expression of adult astroglial or neuronal gene markers (e.g., GFAP, NeuN, Olig2, FoxA2, MAP2), and down-regulation of the HSPC markers CD34 and Runx1. Interestingly, there was higher activation of the pluripotent gene Oct4 in these RFP+YFP+ hybrids, as well as of neuronal precursor genes, such as Sox2 and Nestin, with respect to that observed in the unfused (RFP+) cells (Figure 3E). In contrast, endoderm and mesoderm lineage markers were not up-regulated in the RFP+YFP+ cells, which overall suggests that the hybrids commit toward neuroectoderm differentiation (Figure 3E).

Next, to further investigate the reprogrammed phenotype of the hybrids genome-wide, the inventors analyzed the transcriptome of the RFP+YFP+ cells compared to the RFP+ unfused cells using microarrays. A large number of genes were up-regulated in the RFP+YFP+ hybrids with respect to the RFP+ cells. Gene ontology (GO) analysis of the up-regulated genes showed a statistically significant enrichment of genes associated with the GO term tissue morphogenesis only in the hybrids. Overexpression of this set of genes is indicative of a reprogramming process and is in line with the emerging concept of *in vivo* neuronal reprogramming. Furthermore, gene-set enrichment analysis (Subramanian et al., 2005) showed that hematopoietic stem and progenitor genes were significantly down-regulated in the RFP+YFP+ hybrids (FDR<25%), while neurogenesis genes were enriched, further indicating reprogramming toward a neuronal lineage.

Overall these data showed that the hybrids that formed *in vivo* undergo reprogramming and morphological changes by 1 week after HSPC transplantation.

### Example 4- Hybrids acquire astroglia features and survive over the long-term after transplantation

Four weeks after the intracerebral transplantation, in the MPTP-treated mice, RFP+YFP+ cells located in the ventral midbrain near the SNpc exhibited a morphology typical of glial cells. They did not participate in scar formation, as they were not located in the needle track nor in the transplantation site. These glial cells showed highly branched fine processes that often contacted the basement membrane that surrounds blood vessels. In the MPTP-treated mice, there were more RFP+ cells than in the Saline-treated mice, and 4.75±1.61% of the RFP+ cells were also YFP+ (Figure 4A, Figure 4G). Glial cells that are positive only for RFP suggest possible transdifferentiation from the unfused HSPC-transplanted cells, or silencing of the YFP transgene in the hybrids. Furthermore, the inventors also observed a few YFP+ (RFP-) cells, which were probably hybrids that had switched off the RFP transgene.

Most of the RFP+ and YFP+ cells partially co-expressed the astroglial marker GFAP. None of the highly branched RFP+ and YFP+ cells co-expressed the microglial marker CD11b or the neuronal marker NeuN. Nevertheless, the inventors observed some RFP+ cells with a smaller and less-branched shape that showed partial co-localization with CD11b. Thus, by 4 weeks after transplantation, the RFP+YFP+ cells had mainly acquired properties of mature astroglial cells, and did not express neuronal markers.

The inventors further validated whether the hybrids after fusion of HSPCs with glial cells acquired features of mature astroglia over the long-term. Thus, they grafted HSPCs^{R26Y} into MPTP-treated GFAP-Cre mice, which express Cre only in astroglia (Figure 4B). Four weeks after transplantation, they observed that YFP+ cells localized in the diseased ventral midbrain associated with GFAP+ astroglial cells (Figure 4C). Their levels were 5.42±0.38% with respect to the total GFAP+ cell population. Interestingly, this percentage was considerably high considering that after transplantation they were 1.52±0.37%(Figure 4D). Most of the YFP+ cells co-expressed GFAP (89.75±6.19%) or the mature astrocyte-specific cytosolic protein S100β (Figure 4E,F). Moreover, some cells also expressed the neuroprogenitor marker Sox2, which suggests that some of the hybrids were reprogrammed. Instead, they did not co-express oligodendrocyte transcription factor 2 (Olig2) or microglia marker lba-1. There were low levels of YFP-positive cells that were not astroglial cells (10.25% ±6.19%; Figure 4F), although they could not identify their nature through marker analysis.

The inventors found GFAP+YFP+ hybrids 4 weeks after transplantation also in the 6OHDA Parkinson's disease model. The YFP+ cells co-localized with Cre in the sections from both MPTP- and 6OHDA-transplanted mice, further confirming that the YFP+ cells were indeed hybrids.

All of these data indicate that only the hybrids derived from the fusion of transplanted HSPCs with glial cells, or the hybrids that acquire astroglia features over the long-term, survive up to 4 weeks after the surgery. To further demonstrate this, the inventors grafted HSPCs^{R26Y} into MPTP-treated FoxA2-Cre mice, to obtain neuron-derived hybrids to follow over the long-term after transplantation (Figure 4H). The inventors could not find YFP+ cells with astroglial or neuronal morphologies 4 weeks after transplantation, but only at early time points, further suggesting that neuron-derived hybrids do not survive. In addition, the inventors FACS-sorted RFP+YFP+ hybrids and RFP+ cells (obtained after transplanting HSPCs^{R26Y} in MPTP-treated GFAP-Cre or in MPTP-treated FoxA2-Cre mice) and analyzed their transcriptome profile by microarray analysis. Of note, the RFP+YFP+ hybrids obtained from the GFAP-Cre mice were analyzed 7 days after transplantation. Instead the RFP+YFP+ hybrids obtained from the FoxA2-Cre mice were analyzed 5 days after transplantation, as their numbers at 7 days after transplantation were already very low and were not sufficient to perform genome-wide analysis. The inventors performed gene-set enrichment analysis on the up-regulated genes in the hybrids, and observed that the RFP+YFP+ hybrids obtained from the GFAP-Cre mice were more significantly enriched (FDR < 25%) in neurogenesis genes than those obtained from the FoxA2-Cre mice, further indicating that glial-derived hybrids undergo reprogramming and survive longer. These results are also in agreement with the observation that the RFP+YFP+ hybrids obtained from the R26Y mice display enrichment in neurogenesis genes albeit with high FDR, since they include glial- and neuron-derived hybrids. Overall, these data indicate that hybrids formed by HSPCs with neurons appear not to survive over the long-term, as opposed to the glial-derived hybrids.

### Example 5- Activation of the Wnt signaling pathway is essential for Parkinson's disease rescue after HSPC transplantation

The Wnt signaling pathway has a key role in neuronal cell survival and homeostasis. Moreover, MPTP treatment itself induces temporal and dynamic changes in Wnt signaling components during dopaminergic degeneration. Consequently, the inventors investigated the changes in Wnt signaling after HSPC transplantation in MPTP-injured mice. They dissected out the ventral midbrain area of transplanted MPTP-treated and saline-treated mice, and observed that the protein levels of the transcriptionally active form of β-catenin underwent significant up-regulation in HSPC-transplanted mice when the lesion was stabilized (Vila et al., 2001) (Figure 5G,H). Accordingly, the direct Wnt/β-catenin targets Axin2 and the canonical Wnt pathway receptor Fzd1 were up-regulated (Figure 5I). In contrast, Wnt5a, which is a regulator of the noncanonical pathway, did not change (Figure 5I). Interestingly, GFAP mRNA (Figure 5J) and protein (Figure 5K) also increased, which suggests that activation of astroglial cells in the midbrain of the transplanted mice correlates with activation of Wnt signaling after HSPC transplantation. Finally, they observed reduced levels of Caspase-9 expression in the HSPC-transplanted mice, which was comparable to the level in the Saline group, thus indicating reduced apoptosis in the treated mice (Figure 5L).

Astroglial cells produce Wnt1, which has been shown to have a trophic effect on neighboring neurons *in vitro.* The authors confirmed secretion of Wnt1 by astroglia *in vivo* along the caudal and rostral aqueduct, which is an area that is enriched in astroglia; the rostral area is closest to the transplantation site in the ventral midbrain. Interestingly, Wnt1 was internalized by the nearby neurons (Figure 5M). With this in mind, the inventors analyzed and observed that glial-derived hybrids also secrete Wnt1 (Figure 5A), which suggests its protective effect on the surrounding dopaminergic neurons. Wnt1 transcript and protein levels were significantly increased in the ventral midbrain after MPTP administration and HSPC transplantation, with respect to the Saline and Sham controls (Figure 5B, C, Figure 5N), which indicates increased Wnt1 secretion, which is probably dependent on the hybrids.

As the presence of hybrid-derived astroglia correlated with the observed functional rescue of Parkinson's disease, and at the same time the inventors observed activation of the Wnt/β-catenin canonical pathway in the transplanted area and secretion of Wnt1 by the hybrids, they aimed to address the effect of the block of Wnt/β-catenin signaling activity in the HSPC-transplanted mice. Remarkably, treatment with the Wnt antagonist Dkk1 (unilateral infusion of 2 µg/µl in the SNpc) impaired the recovery of dopaminergic neurons mediated by HSPCs in both the MPTP and 6OHDA models (Figure 1C, E), which suggests that activation of the pathway is essential for the observed rescue phenotype.

As a final demonstration that ablation of the hybrids can impair the rescue, the inventors used transgenic mice carrying the Rosa26-LoxP-STOP-LoxP-DTR (R26-diphtheria toxin receptor) and transplanted these with HSPCs^{Cre/RFP} (Figure 5D). After fusion, the hybrids expressed both RFP and DTR, making them sensitive to diphtheria toxin (DT) injection; thus, they were selectively ablated (Figure 5O,P). Four weeks after transplantation, the percentages of TH+ fibers and TH+ neurons were very low in both the Sham and HSPC-transplanted mice that received the toxin (Figure 5E, F), which indicates that ablation of the hybrids impairs the observed rescue.

### Example 6-Effect of transplantation of HSPCs having the Wnt/β-catenin pathway activated

Aging, the strongest risk factor for Parkinson's disease's, and the 1-methyl-4-phenyl-1,2,3,6-tetrahydropyridine (MPTP) mouse model of Parkinson's disease are associated with dysregulation of Wnt1/β-catenin signaling and gradual decline of the capacity of dopaminergic neurons to recover upon injury (L'episcopo et al., 2010). Activation of Wnt signaling triggers cell-fusion-mediated reprogramming of neural precursor cells in culture and *in vivo* (Lluis et al., 2008, 2011, Sanges et al., 2013). With this in mind, the inventors tested whether HSPCs transplantation after Wnt/β-catenin pathway activation promotes dopaminergic neurons recovery. Untreated HSPCs and HSPCs in which the Wnt signaling pathway had been previously activated by the GSK-3 inhibitor 6-bromoindirubin-30-oxime (BIO) with 3 mM BIO, for 24 hours were stereotaxically transplanted into the middle-aged (12 month) mice. Approximately 6x10⁴ cells of non-treated HSPCs or BIO-pretreated were transplanted in the MPTP treated mice.

Using an unbiased stereology system, the inventors then quantified the number of TH+ immunoreactive cells in the SNpc and the density of TH+ fibers in the striatum, by optical densitometry. Four weeks after transplantation, the percentage of TH+ cells in the sham-transplanted MPTP-lesioned mice remained very low bilaterally, with up to a 40.41±5.71% loss of DA neurons and up to 35.15±1.54% loss of fibers, in comparison with saline-treated animals (Figure 6). Interestingly, in the group of transplanted MPTP-lesioned mice with HSPCs (BIO treated), the loss of TH+ cells in the SNpc was only around 19.13±4.82%, and the density of TH+ fibers in the striatum was around 26.48±3.69%, compared to saline-treated animals. Thus, there was significant reduction in MPTP-induced loss of dopaminergic neurons in transplanted mice with HSPC (BIO treated), suggesting that the activation of the Wnt1/β-catenin signaling increases efficacy in aged mice.

### BIBLIOGRAPHY

Acquistapace, A., Bru, T., Lesault, P.F., Figeac, F., Coudert, A.E., le Coz, O., Christov, C., Baudin, X., Auber, F., et al. (2011). Stem Cells 29, 812-824.
Amamoto, R., and Arlotta, P. (2014). Science 343, 1239882.
Bar-Nur, O., Verheul, C., Sommer, A.G., Brumbaugh, J., Schwarz, B.A., Lipchina, I., Huebner, A.J., Mostoslavsky, G., and Hochedlinger, K. (2015). Nat Biotechnol 33, 761-68.
Buch, T., Heppner, F.L., Tertilt, C., Heinen, T.J., Kremer, M., Wunderlich, F.T., Jung, S., and Waisman, A. (2005). Nat Methods 2, 419-426.
Bortolozzi, A. and Artigas, F. (2003). Neuropsychopharmacol 28, 421-434.
Chen, D.C., Lin, S.Z., Fan, J.R., Lin, C.H., Lee, W., Lin, C.C., Liu, Y.J., Tsai, C.H., Chen, J.C., et al. (2014). Cell Transplant 23, 1599-1612.
Gaillard, A., and Jaber, M. (2011). Trends Neurosci 34, 124-133.
Gregorian, C., Nakashima, J., Le Belle, J., Ohab, J., Kim, R., Liu, A., Smith, K.B., Groszer, M., Garcia, A.D., Sofroniew, M.V., Carmichael, S.T., Kornblum, H.I., Liu, X., and Wu, H. (2009). J Neurosci 29, 1874-1886.
Hayashi, S., and McMahon, A.P. (2002). Dev Biol 244, 305-318.
Inestrosa, N.C., and Arenas, E. (2010). Nat Rev Neurosci 11, 77-86.
de Jong, J.H., Rodermond, H.M., Zimberlin, C.D., Lascano, V., De Sousa E Melo, F., Richel, D.J., Medema, J.P., and Vermeulen, L. (2012). Sci Rep 2, 271.
Kang, X., Xu, H., Teng, S., Zhang, X., Deng, Z., Zhou, L., Zuo, P., Liu, B., Liu, B., et al. (2014). Proc Natl Acad Sci U S A 111, 15804-09.
Keshet, G.I., Tolwani, R.J., Trejo, A., Kraft, P., Doyonnas, R., Clayberger, C., Weimann, J.M., and Blau, H.M. (2007). J Comp Neurol 504, 690-701.
L'episcopo, F., Serapide, M.F., Tirolo, C., Testa, N., Caniglia, S., Morale, M.C., Pluchino, S., and Marchetti, B. (2011). Mol Neurodegener 6, 49.
L'episcopo, F., Tirolo, C., Testa, N., Caniglia, S., Morale, M.C., Cossetti, C., D'Adamo, P., Zardini, E., Andreoni, L., et al. (2010). Neurobiol Dis 41, 508-527.
L'episcopo, F., Tirolo, C., Testa, N., Caniglia, S., Morale, M.C., Serapide, M.F., Pluchino, S., and Marchetti, B. (2014). Stem Cells 32, 2147-163.
Lluis, F., Pedone, E., Pepe, S. & Cosma, M.P., 2008, Cell stem cell, 3(5), pp. 493-507.
Lluis, F., and Cosma, M.P. (2010). J Cell Physiol 223, 6-13.
Lluis, F., Ombrato, L., Pedone, E., Pepe, S., Merrill, B.J. & Cosma, M.P., 2011, Proceedings of the National Academy of Sciences of the United States of America, 108(29), pp. 11912-7.
Long, J.Z., Lackan, C.S., and Hadjantonakis, A.K. (2005). BMC Biotechnol 5,20.
Marchetti, B., L'Episcopo, F., Morale, M.C., Tirolo, C., Testa, N., Caniglia, S., Serapide, M.F., and Pluchino, S. (2013). Eur J Neurosci 37, 1550-563.
Ogle, B.M., Cascalho, M., and Platt, J.L. (2005). Nat Rev Mol Cell Biol 6, 567-575.
Parish, C.L., Finkelstein, D.I., Drago, J., Borrelli, E., and Horne, M.K. (2001). J Neurosci 21, 5147-157.
Park, S.S., Bauer, G., Abedi, M., Pontow, S., Panorgias, A., Jonnal, R., Zawadzki, R.J., Werner, J.S., and Nolta, J. (2015). Invest Ophthalmol Vis Sci 56, 81-89.
Park, E.J., Sun, X., Nichol, P., Saijoh, Y., Martin, J.F., and Moon, A.M. (2008). Dev Dyn 237, 447-453.
Paxinos, G., and Franklin, K.B.J. (2008). The Mouse Brain in Stereotaxic Coordinates (Elsevier Academic Press).
de Rijk, M.C., Tzourio, C., Breteler, M.M., Dartigues, J.F., Amaducci, L., Lopez-Pousa, S., Manubens-Bertran, J.M., Alpérovitch, A., and Rocca, W.A. (1997). J Neurol Neurosurg Psychiatry 62, 10-15.
Ritchie ME, Silver J, Oshlack A, Silver J, Holmes M, Diyagama D, Holloway A, Smyth GK. (2007). Bioinformatics, 23, 2700-2707.
Sanges, Romo, Simonte, Di Vicino, Diaz Tahoces, Fernández, and Cosma (2013). Cell Rep 4, 1-16.
Schallert, T., Fleming, S.M., Leasure, J.L., Tillerson, J.L., and Bland, S.T. (2000). Neuropharmacology 39, 777-787.
Srinivas, S., Watanabe, T., Lin, C.S., William, C.M., Tanabe, Y., Jessell, T.M., and Costantini, F. (2001). BMC Dev Biol 1, 4.
Subramanian, A., Tamayo, P., Mootha, V.K., Mukherjee, S., Ebert, B.L., Gillette, M.A., Paulovich, A., Pomeroy, S.L., Golub, T.R., et al. (2005). Proc Natl Acad Sci U S A 102, 15545-550.
Vila, M., Jackson-Lewis, V., Vukosavic, S., Djaldetti, R., Liberatore, G., Offen, D., Korsmeyer, S.J., and Przedborski, S. (2001). Proc Natl Acad Sci U S A 98, 2837-842.
Yang, F., Liu, Y., Tu, J., Wan, J., Zhang, J., Wu, B., Chen, S., Zhou, J., Mu, Y., and Wang, L. (2014). Nat Commun 5, 5627.
Zhang, J., Götz, S., Vogt Weisenhorn, D.M., Simeone, A., Wurst, W., and Prakash, N. (2015). Neurobiol Dis 82, 32-45.

## Claims

1. A cell selected from the group consisting of a hematopoietic stem cell (HSC), a hematopoietic progenitor cell (HPC), and a mesenchymal stem cell (MSC), or a cell population comprising any combination thereof, for use in the treatment of Parkinsonism in a subject.

2. The cell population for use according to claim 1 comprising a hematopoietic stem cell (HSC) and a hematopoietic progenitor cell (HPC).

3. The cell population for use according to any one of claims 1 or 2, wherein said cell population is CD34+.

4. The cell population for use according to any of claims 2 or 3 further comprising a mesenchymal stem cell (MSC).

5. The cell or the cell population for use according to any of claims 1-4, wherein said cell or cell population is transplanted into the brain of a subject in need of treatment, preferably in the midbrain, more preferably, above the *substantia nigra* pars compacta (SNpc).

6. The cell or the cell population for use according to any of claims 1 to 5, wherein said cell or cell population is transplanted in a region of the brain having the Wnt/β-catenin signaling pathway activated or in a region of the brain wherein the Wnt/β-catenin signaling pathway is activated after transplantation.

7. The cell or the cell population for use according to anyone of claims 1 to 6, wherein the Wnt/β-catenin signalling pathway of said cells is activated.

8. The cell or the cell population for use according to anyone of claims 1 to 7, wherein said treatment further comprises the previous, simultaneous, sequential or separate administration to the subject in need of treatment of a Wnt/β-catenin signalling pathway activator or an inhibitor of a Wnt/β-catenin signalling pathway repressor.

9. The cell or the cell population for use according to anyone of claims 1 to 8, wherein said cells are pretreated with a Wnt/β-catenin signalling pathway activator, and/or with an inhibitor of a Wnt/β-catenin signalling pathway repressor, and/or said cells overexpress a Wnt/β-catenin signalling pathway activator and/or said cells overexpress an inhibitor of the Wnt/β-catenin signalling pathway repressor.

10. The cell or the cell population for use according to anyone of claims 8 or 9, wherein said Wnt/β-catenin signaling pathway activator is selected from the group consisting of a Wnt isoform, β-catenin, a R-spondin, 2-(4-acetylphenylazo)-2-(3,3-dimethyl-3,4-dihydro-2H-isoquinolin-1-ylidene)-acetamide (IQ1), (2*S*)-2-[2-(indan-5-yloxy)-9-(1,1'-biphenyl-4-yl)methyl)-9*H*-purin-6-ylamino]-3-phenyl-propan-1-ol (QS11), deoxycholic acid (DCA), 2-amino-4-[3,4-(methylenedioxy)benzylamino]-6-(3-methoxyphenyl)pyrimidine,
an (hetero)arylpyrimidine of formula (I) 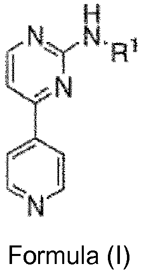 wherein R¹ is N-(3-1-H-imidazol-1-yl)propane, N-(2-pyridin-4-yl)ethane, N-(2-pridin- 3-yl)ethane, N-(3-(3,5-dimethyl-1H-pyrazol-1-yl)propyl, N-(2-(1-H-indol-3-yl)ethane, or N-(S)-3-1 H-indol-3-yl)-2-propan-1-ol amine;
an (hetero)arylpyrimidine of formula (II), 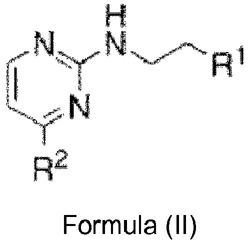 wherein R¹ is CH₂-1H-imidazole, 4-pyridine, 3-(1H-indole), 3-(2-methyl-1H-indol-5-ol, or 4-(1-H-imidazole), and R² is 4-(pyridine-4-yl), 4-(pyridine-3-yl), 4-(3-nitrophenyl), 2- (benzo[b]thiophene) or 2-(naphthyl),
an (hetero)arylpyrimidine of formula (III), 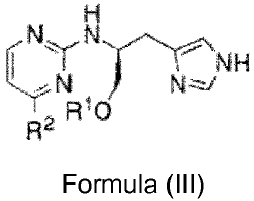 wherein R¹ is H, ethyl, methylenecyclohexyl, 2-fluoro-3-(trifluoromethyl)benzyl or prop- 2-ynyl, and R² is 2-(benzo[b]thiophene) or 2-(naphthyl),
an (hetero)arylpyrimidine of formula (IV) 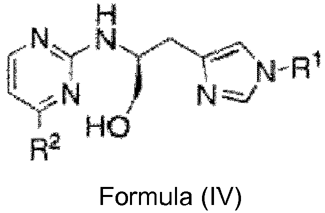 wherein R¹ is 3,5-difluorobenzyl, prop-2-ynyl, 2-acetamide or 3-propanitrile and R² is 2- (benzo[b]thiophene) or 2-(naphthyl),
and combinations thereof.

11. The cell or the cell population for use according to anyone of claims 8 or 9, wherein said inhibitor of a Wnt/β-catenin signaling pathway repressor is selected from the group consisting of WAY-316606, 3-(2,4-dichlorophenyl)-4-(1-methyl-1H-indol-3-yl)-1H-pyrrole-2,5-dione, 3-[(3-chloro-4-hydroxyphenyl)amino]-4-(2-nitro-phenyl)-1H-pyrrole-2,5-dione, BIO (6-bromoindirubin-3'-oxime), BIO-acetoxime, MeBIO, Indirubin-3'-oxime, 5-Ethyl-7,8-dimethoxy-1H-pyrrolo[3,4-c]isoquinoline-1,3(2H)-dione, N-[(4-Methoxyphenyl)methyl]-N'-(5-nitro-2-thiazolyl)urea, 6-[[2-[[4-(2,4-Dichlorophenyl)-5-(5-methyl-1H-imidazol-2-yl)-2-pyrimidinyl]amino]ethyl]amino]-3-pyridinecarbonitrile, 6-[[2-[[4-(2,4-Dichlorophenyl)-5-(5-methyl-1H-imidazol-2-yl)-2-pyrimidinyl]amino]ethyl]amino]-3-pyridinecarbonitrile trihydrochloride, Lithium carbonate, 3-[[6-(3-Aminophenyl)-7H-pyrrolo[2,3-d]pyrimidin-4-yl]oxyphenol ditrifluoroacetate, 3-[5-[4-(2-Hydroxy-2-methyl-1-oxopropyl)-1-piperazinyl]-2-(trifluoromethyl)phenyl]-4-(1H-indol-3-yl)-1H-pyrrole-2,5-dione, 2-Methyl-5-[3-[4-(methylsulfinyl)phenyl]-5-benzofuranyl]-1,3,4-oxadiazole, and combinations thereof.

12. The cell or the cell population for use according to anyone of claims 1 to 11 wherein said cells, after transplantation into the brain of the subject, are fused with a neuron or with a glial cell from said subject, preferably with a glial cell, whereby a hybrid cell is formed and reprogramming of said hybrid cell is induced.

13. A pharmaceutical composition selected from the group consisting of:
1) a pharmaceutical composition comprising a cell selected from the group consisting of a hematopoietic stem cell (HSC), a hematopoietic progenitor cell (HPC), and a mesenchymal stem cell (MSC), or a cell population comprising any combination thereof, and a pharmaceutically acceptable carrier;
2) a pharmaceutical composition comprising a cell selected from the group consisting of a hematopoietic stem cell (HSC), a hematopoietic progenitor cell (HPC), and a mesenchymal stem cell (MSC), or a cell population comprising any combination thereof, wherein the Wnt/β-catenin signaling pathway of said cells is activated, and a pharmaceutically acceptable carrier; and
3) a pharmaceutical composition comprising a cell selected from the group consisting of a hematopoietic stem cell (HSC), a hematopoietic progenitor cell (HPC), and a mesenchymal stem cell (MSC), or a cell population comprising any combination thereof, a Wnt/β-catenin signalling pathway activator or an inhibitor of a Wnt/β-catenin signalling pathway repressor, and a pharmaceutically acceptable carrier,
for use in the treatment of Parkinsonism.

14. The pharmaceutical composition for use in the treatment of Parkinsonism according to claim 13, wherein said treatment comprises transplantation of said pharmaceutical composition into the brain, preferably into the midbrain, more preferably, above the *substantia nigra* pars compacta (SNpc), of a subject in need of treatment.

15. The pharmaceutical composition for use according to anyone of claims 13 or 14, wherein the cell population contained in said pharmaceutical composition comprises a hematopoietic stem cell (HSC) and a hematopoietic progenitor cell (HPC).
